# NEW EUROPEAN PATENT SPECIFICATION

(11) **EP 2 750 630 B2**
(45) Date of publication and mention of the opposition decision: **02.12.2026**
(45) Mention of the grant of the patent: 30.06.2021
(21) Application number: 12784809.1
(22) Date of filing: 19.10.2012
(51) Int. Cl.: A61F 2/24

(54) **DEVICE FOR HEART VALVE REPLACEMENT**
VORRICHTUNG FÜR DEN ERSATZ EINER HERZKLAPPE
DISPOSITIF DE REMPLACEMENT DE VALVULE CARDIAQUE

(30) Priority: 19.10.2011 US 201161549037 P
(43) Date of publication of application: 09.07.2014
(60) Divisional application: 21181385.2 / 3 943 047; 23192834.2 / 4 252 714; 25192909.7 / 4 616 829; 26175682.9 / 4 763 142
(73) Proprietor: Twelve, Inc., Menlo Park, CA 94025 (US)
(72) Inventor: MORRISS, John, San Francisco, CA 94107 (US); GIFFORD, Hanson, Woodside, CA 94062 (US); FANN, James, L., Portola Valley, CA 94028 (US); DUERI, Jean-Pierre, Los Gatos, CA 95032 (US); GITTINGS, Darrin, Sunnyvale, CA 94086 (US); LUNA, Michael, San Jose, CA 95123 (US); DEEM, Mark, Mountain View, CA 94041 (US); SUTTON, Douglas, Pacifica, CA (US)
(74) Representative: Zimmermann & Partner Patentanwälte mbB
(86) International application number: PCT/US2012/061215
(87) International publication number: WO 2013/059743

(56) References cited:
- WO-A1-2010/098857
- WO-A1-2011/051043
- WO-A2-2011/072084
- US-A1- 2006 149 360
- US-A1- 2008 082 166
- US-A1- 2009 198 315
- US-A1- 2010 249 915
- US-A1- 2010 298 931
- US-A1- 2010 312 333
- US-A1- 2011 029 072
- US-A1- 2011 172 784

## Description

### TECHNICAL FIELD

The present technology relates generally to prosthetic heart valve devices. In particular, several embodiments are directed to prosthetic mitral valves and devices for percutaneous repair and/or replacement of native heart valves and associated systems and methods.

### BACKGROUND

Conditions affecting the proper functioning of the mitral valve include, for example, mitral valve regurgitation, mitral valve prolapse and mitral valve stenosis. Mitral valve regurgitation is a disorder of the heart in which the leaflets of the mitral valve fail to coapt into apposition at peak contraction pressures, resulting in abnormal leaking of blood from the left ventricle into the left atrium. There are a number of structural factors that may affect the proper closure of the mitral valve leaflets. For example, many patients suffering from heart disease experience dilation of the heart muscle, resulting in an enlarged mitral annulus. Enlargement of the mitral annulus makes it difficult for the leaflets to coapt during systole. A stretch or tear in the chordae tendineae, the tendons connecting the papillary muscles to the inferior side of the mitral valve leaflets, may also affect proper closure of the mitral annulus. A ruptured chordae tendineae, for example, may cause a valve leaflet to prolapse into the left atrium due to inadequate tension on the leaflet. Abnormal backflow can also occur when the functioning of the papillary muscles is compromised, for example, due to ischemia. As the left ventricle contracts during systole, the affected papillary muscles do not contract sufficiently to effect proper closure.

Mitral valve prolapse, or when the mitral leaflets bulge abnormally up in to the left atrium, causes irregular behavior of the mitral valve and may also lead to mitral valve regurgitation. Normal functioning of the mitral valve may also be affected by mitral valve stenosis, or a narrowing of the mitral valve orifice, which causes impedance of filling of the left ventricle in diastole.

Typically, treatment for mitral valve regurgitation has involved the application of diuretics and/or vasodilators to reduce the amount of blood flowing back into the left atrium. Other procedures have involved surgical approaches (open and intravascular) for either the repair or replacement of the valve. For example, typical repair approaches have involved cinching or resecting portions of the dilated annulus.

Cinching of the annulus has been accomplished by the implantation of annular or peri-annular rings which are generally secured to the annulus or surrounding tissue. Other repair procedures have also involved suturing or clipping of the valve leaflets into partial apposition with one another.

Alternatively, more invasive procedures have involved the replacement of the entire valve itself where mechanical valves or biological tissue are implanted into the heart in place of the mitral valve. These are conventionally done through large open thoracotomies and are thus very painful, have significant morbidity, and require long recovery periods.

However, with many repair and replacement procedures the durability of the devices or improper sizing of annuloplasty rings or replacement valves may result in additional problems for the patient. Moreover, many of the repair procedures are highly dependent upon the skill of the cardiac surgeon where poorly or inaccurately placed sutures may affect the success of procedures.

Less invasive approaches to aortic valve replacement have been developed in recent years. Examples of pre-assembled, percutaneous prosthetic valves include, e.g., the CoreValve Revalving^{®} System from Medtronic/Corevalve Inc. (Irvine, CA, USA) the Edwards-Sapien^{®} Valve from Edwards Lifesciences (Irvine, CA, USA). Both valve systems include an expandable frame housing a tri-leaflet bioprosthetic valve. The frame is expanded to fit the substantially symmetric circular aortic valve. This gives the expandable frame in the delivery configuration a symmetric, circular shape at the aortic valve annulus, perfectly functional to support a tri-leaflet prosthetic valve (which requires such symmetry for proper coaptation of the prosthetic leaflets). Thus, aortic valve anatomy lends itself to an expandable frame housing a replacement valve since the aortic valve anatomy is substantially uniform and symmetric. The mitral valve, on the other hand, is generally D-shaped and not symmetric, meaning that expansion of the CoreValve and Sapien systems in the mitral valve renders such systems non-functional. For example, in both systems the frame both anchors (or helps to anchor) and provides shape to the replacement valve within. If the frame is flexible enough to assume the asymmetric shape of the mitral valve, then the attached tri-leaflet replacement valve will also be similarly shaped, making it almost impossible for the leaflets to coapt properly and thus allowing leaks. Additionally, if the frame is so rigid that it remains symmetric, the outer diameter of the frame will not be able to cover the commissures of the mitral valve, also allowing leaks.

In addition, mitral valve replacement, compared with aortic valve replacement, poses unique anatomical obstacles, rendering percutaneous mitral valve replacement significantly more involved and challenging than aortic valve replacement. First, unlike the relatively symmetric and uniform aortic valve, the mitral valve annulus has a non-circular D-shape or kidney-like shape and may be of unpredictable geometry, often times lacking symmetry. Such unpredictability makes it difficult to design a mitral valve prosthesis having the ability to conform to the mitral annulus. Lack of a snug fit between the leaflets and/or annulus and the prosthesis leaves gaps therein, creating backflow of blood through these gaps. Placement of a cylindrical valve prosthesis, for example, may leave gaps in commissural regions of the native valve, potentially resulting in perivalvular leaks in those regions.

Current devices seeking to overcome the large and irregular shape of the mitral valve have several drawbacks. First, many of the devices today have a direct, structural connection between the device structure which contacts the annulus and/or leaflets and the device structure which supports the prosthetic valve. In several devices, the same stent posts which support the prosthetic valve also contact subannular tissue, directly transferring many of the distorting forces present in the heart, for example, systolic pressure, diastolic pressure, compressive intra-annular forces, etc., causing hoop stress in the stent portion surrounding the prosthetic valve. Most cardiac replacement devices utilize a tri-leaflet valve, which requires a substantially symmetric, cylindrical support around the prosthetic valve for proper opening and closing of the three leaflets. Devices which provide a direct, mechanical connection between annular and/or leaflet distorting forces and the prosthetic valve may compress and/or distort the symmetrical, cylindrical structure surrounding the prosthetic valve causing the prosthetic leaflets to malfunction.

In addition to its irregular, unpredictable shape, the mitral valve annulus lacks a significant amount of radial support from surrounding tissue. The aortic valve, for example, is completely surrounded by fibro-elastic tissue, helping to anchor a prosthetic valve by providing native structural support. The mitral valve, on the other hand, is bound by muscular tissue on the outer wall only. The inner wall of the mitral valve is bound by a thin vessel wall separating the mitral valve annulus from the inferior portion of the aortic outflow tract. As a result, significant radial forces on the mitral annulus, such as that imparted by expanding stent prostheses, could lead to collapse of the inferior portion of the aortic tract with potentially fatal consequences.

The chordae tendineae of the left ventricle may also present an obstacle in deploying a mitral valve prosthesis. This is unique to the mitral valve since aortic valve anatomy does not include chordae. The maze of chordae in the left ventricle makes navigating and positioning a deployment catheter more difficult in mitral valve replacement and repair. Deployment and positioning of a prosthetic valve or anchoring device on the ventricular side of the native valve is also complicated by the presence of the chordae.

Given the difficulties associated with current procedures, there remains the need for simple, effective, and less invasive devices and methods for treating dysfunctional heart valves.

### PRIOR ART

US patent application 2010/031233 A1 describes an apparatus for replacing a native cardiac valve. The native cardiac valve has at least one leaflet and is sur- rounded by a native cardiac valve annulus having superior and inferior aspects. The apparatus comprises a barbell- shaped, expandable anchoring member including first, second, and main body portions extending between the end portions. The main body portion includes a channel defined by inner and outer surfaces. Each of the first and second end portions has a diameter greater than the diameter of the main body portion. The first and second end portions are sized to respectively contact the superior and inferior aspects of the native cardiac valve annulus when the expandable anchoring member is in an expanded configuration. The apparatus also includes an expandable support member operably disposed within the main body portion of the expandable anchoring member, and a prosthetic cardiac valve secured within the expandable support member.

US patent application 2006/0149360 A1 describes a prosthetic device including a valve-orifice attachment member attachable to a valve in a blood vessel and including a fluid inlet, and a diverging member that extends from the fluid inlet, the diverging member including a proximal end near the fluid inlet and a distal end distanced from the proximal end, wherein a distal portion of the diverging member has a larger cross-sectional area for fluid flow therethrough than a proximal portion thereof. The diverging member may have a diverging taper that causes fluid to flow therethrough with pressure recovery at the distal end thereof.

US patent application 2009/0198315 A1 describes medical implants/stents, systems, and methods of treatment using such stents. Some embodiments of the invention include a self-expanding stent having a tubular first wall structure of a first diameter, where each end portion of the first wall structure is formed into a second wall structure of a second diameter larger than the first diameter. A membrane covers at least the first wall structure and the stent includes an interlaced, helicoidally wound wire forming a mesh-like structure.

Further relevant prior art is disclosed by US 2011/029072 A1.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure. Furthermore, components can be shown as transparent in certain views for clarity of illustration only and not to indicate that the illustrated component is necessarily transparent.
Figures 1 and 2 are schematic illustrations of a mammalian heart having native valve structures suitable for replacement with various prosthetic heart valve devices in accordance with embodiments of the present technology.
Figure 3 is a schematic cross-sectional side view of a native mitral valve showing the annulus and leaflets.
Figure 4A is a schematic illustration of the left ventricle of a heart having either i) prolapsed leaflets in the mitral valve, or ii) mitral valve regurgitation in the left ventricle of a heart having impaired papillary muscles, and which are suitable for combination with various prosthetic heart valve devices in accordance with embodiments of the present technology.
Figure 4B is a schematic illustration of a heart in a patient suffering from cardiomyopathy, and which is suitable for combination with various prosthetic heart valve devices in accordance with embodiments of the present technology..
Figures 5A is a schematic illustration of a native mitral valve of a heart showing normal closure of native mitral valve leaflets.
Figure 5B is a schematic illustration of a native mitral valve of a heart showing abnormal closure of native mitral valve leaflets in a dilated heart, and which is suitable for combination with various prosthetic heart valve devices in accordance with embodiments of the present technology.
Figure 5C is a schematic illustration of a mitral valve of a heart showing dimensions of the annulus, and which is suitable for combination with various prosthetic heart valve devices in accordance with embodiments of the present technology.
Figure 6A is a schematic, cross-sectional illustration of the heart showing an antegrade approach to the native mitral valve from the venous vasculature in accordance with various embodiments of the present technology.
Figure 6B is a schematic, cross-sectional illustration of the heart showing access through the inter-atrial septum (IAS) maintained by the placement of a guide catheter over a guidewire in accordance with various embodiments of the present technology.
Figures 7 and 8 are schematic, cross-sectional illustrations of the heart showing retrograde approaches to the native mitral valve through the aortic valve and arterial vasculature in accordance with various embodiments of the present technology.
Figure 9 is a schematic, cross-sectional illustration of the heart showing an approach to the native mitral valve using a trans-apical puncture in accordance with various embodiments of the present technology.
Figure 10A shows an isometric view of a prosthetic heart valve device in accordance with an embodiment of the present technology.
Figure 10B illustrates a cut-away view of a heart showing the prosthetic heart valve device of Figure 10A implanted at a native mitral valve in accordance with an embodiment of the present technology.
Figures 10C-10D are side and top views, respectively, of a prosthetic heart valve device in accordance with an embodiment of the present technology.
Figure 11 is an isometric view of a valve support with a prosthetic valve mounted therein in accordance with an embodiment of the present technology.
Figures 12A-12H are side views of various mechanisms of coupling a valve support to a retainer in accordance with additional embodiments of the present technology.
Figures 13A-13G are partial side views of a variety of flexible rib configurations in accordance with additional embodiments of the present technology.
Figures 14A-14J are side views of various flexible ribs flexing in response to a distorting force in accordance with further embodiments of the present technology.
Figures 15A-15E are schematic top views of the prosthetic heart valve device showing a variety of rib configurations in accordance with further embodiments of the present technology.
Figures 16A-16B are schematic side and cross-sectional views of the prosthetic heart valve device showing additional embodiments of the retainer in accordance with the present technology.
Figure 17A is a schematic top view of a native mitral valve illustrating the major and minor axes.
Figures 17B-17C are schematic top views of a retainer in an expanded configuration and in a deployed configuration, respectively, in accordance with an embodiment of the present technology.
Figure 18 is a side view of a prosthetic heart valve device shown in an expanded configuration in accordance with a further embodiment of the present technology.
Figure 19 is an isometric view of the prosthetic heart valve device having a connecting ring in accordance with an embodiment of the present technology.
Figures 20A-20B are isometric views of a retainer support ring and the prosthetic heart valve device having the retainer support ring in accordance with an additional embodiment of the present technology.
Figure 21 is an isometric view of a prosthetic heart valve device in an expanded configuration and having a plurality of stabilizing elements in accordance with an embodiment of the present technology.
Figure 22 is an enlarged schematic, side view of a prosthetic heart valve device having an extended arm in accordance with an embodiment of the present technology.
Figures 23A-23C are enlarged partial side views of a prosthetic heart valve device having arms coupled to the device at various angles with respect to a longitudinal axis of the device in accordance with further embodiments of the present technology.
Figures 24A-24C are enlarged, partial side views of a prosthetic heart valve device having arms of various lengths coupled to the device in accordance with additional embodiments of the present technology.
Figures 25A-25E are cross-sectional views of a heart with an implanted prosthetic heart valve device having arms disposed on an inward-facing surface of the leaflets in accordance with various embodiments of the present technology.
Figures 26A-26C are schematic views illustrating various embodiments of tissue engaging elements for use with prosthetic heart valve devices in accordance with the present technology.
Figures 27A-27C are enlarged, partial side views of a prosthetic heart valve device having arms with tissue engaging elements configured to engage an inward-facing surface of the leaflets in accordance with various embodiments of the present technology.
Figures 28A-28B are side views showing prosthetic heart valve devices implanted at a mitral valve MV (illustrated in cross-section) in a deployed configuration, wherein the devices have arms for engaging an outward-facing surface of the native leaflets in accordance with further embodiments of the present technology.
Figure 28C is an enlarged, partial side view of a prosthetic heart valve device having an arm with tissue engaging elements configured to engage an outward-facing surface of the leaflets in accordance with another embodiment of the present technology.
Figure 29A is a side view of a prosthetic heart valve device and shown implanted at a mitral valve (illustrated in cross-section), the device having arms for engaging an outward-facing surface of the native leaflets and arms for engaging an inward-facing surface of the native leaflets in accordance with an additional embodiment of the present technology.
Figure 29B is an enlarged view of the arms engaging the inward-facing and outward-facing surfaces of the leaflets as shown in Figure 29A.
Figures 30A and 30C are isometric views of the prosthetic heart valve device having arms with a similar profile as a profile of the retainer in accordance with additional embodiments of the present technology.
Figures 30B and 30D are side views of the prosthetic heart valve devices of Figures 30A and 30C, respectively, and shown implanted at a mitral valve (illustrated in cross-section) in accordance with the present technology.
Figure 31A is a side view of a prosthetic heart valve device having a plurality of non-interconnected arms in accordance with a further embodiment of the present technology.
Figure 32B is a side view of a prosthetic heart valve device having a plurality of circumferentially connected arms in accordance with a further embodiment of the present technology.

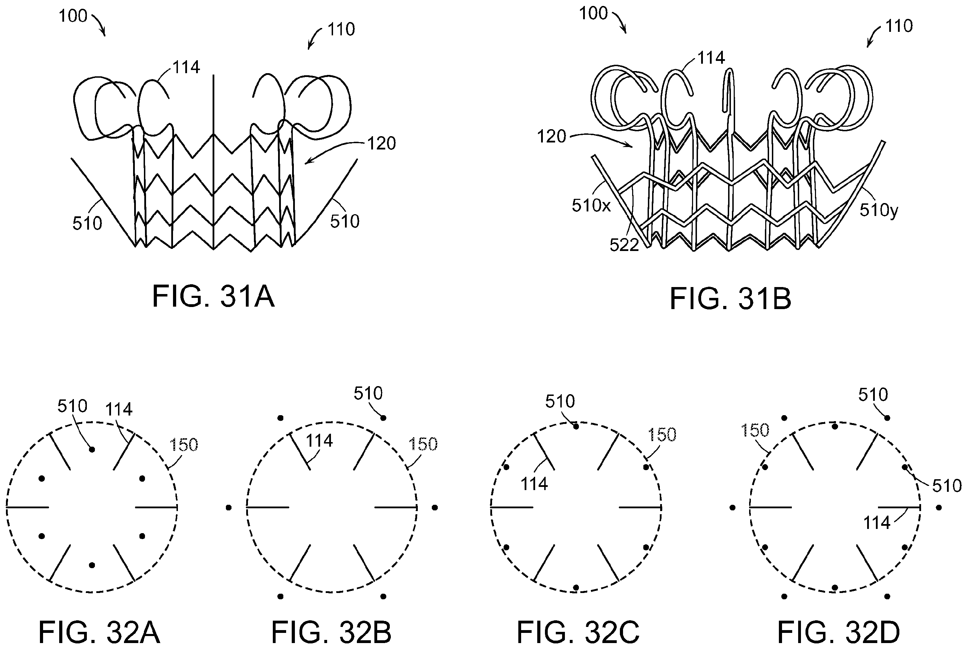

Figures 32A-32D are schematic top views of arm location patterns in accordance with additional embodiments of the present technology.

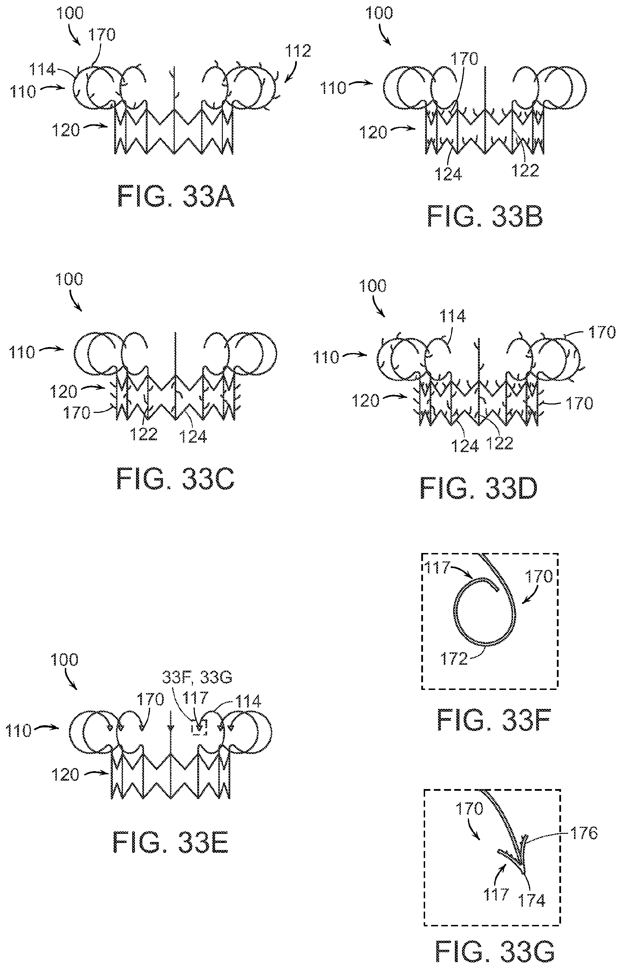

Figures 33A-33E are side views of prosthetic heart valve devices having tissue engaging elements on varying structures of the device in accordance with additional embodiments of the present technology.
Figures 33E-33G are enlarged side views of tissue engaging elements suitable for use with prosthetic heart valve devices in accordance with other embodiments of the present technology.

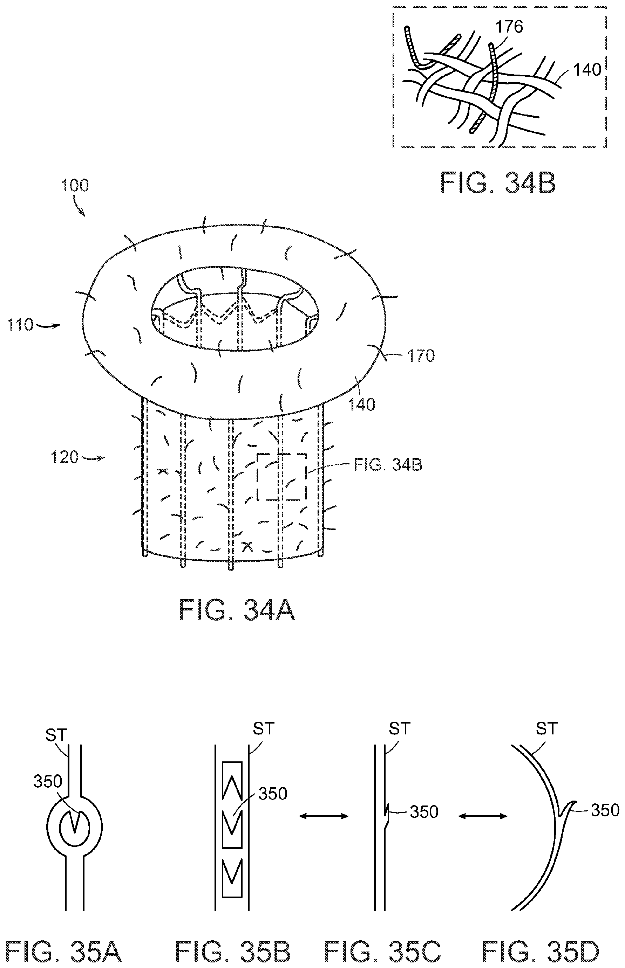

Figures 34A-34B are an isometric view and an enlarged detail view of a prosthetic heart valve device having a sealing member configured with tissue engaging elements in accordance with another embodiment of the present technology
Figures 35A-35F are enlarged side views of embodiments of tissue engaging elements suitable for use with prosthetic heart valve devices in accordance with additional embodiments of the present technology.

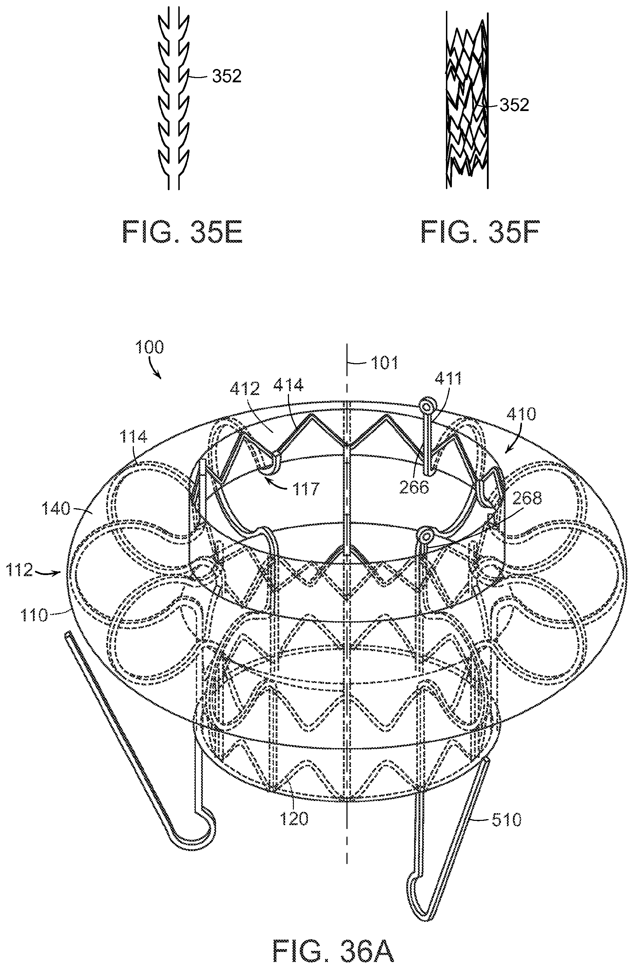

Figure 36A is an isometric view of a prosthetic heart valve device 100 having an atrial extension member 410 in accordance with various embodiments of the present technology.

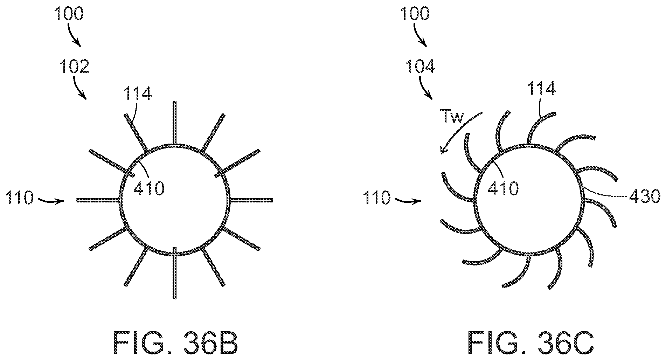

Figures 36B-36C are schematic, top views of an embodiment of a prosthetic heart valve device having an atrial extension member without (Figure 36B) and with (Figure 36C) a twisting force applied to the device in accordance with the present technology.

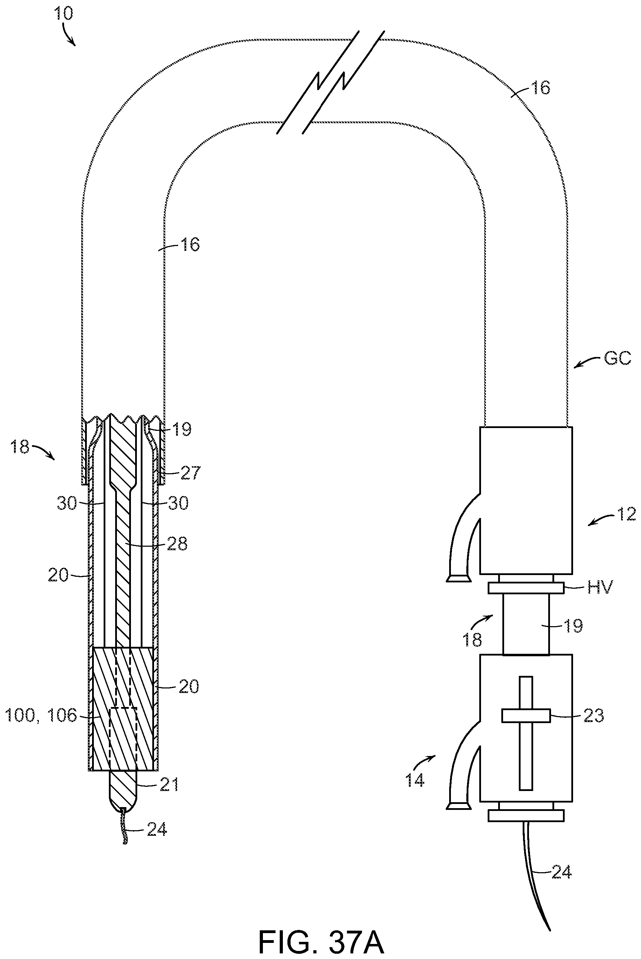

Figure 37A is side partial cut-away view of a delivery system in accordance with an embodiment of the present technology.

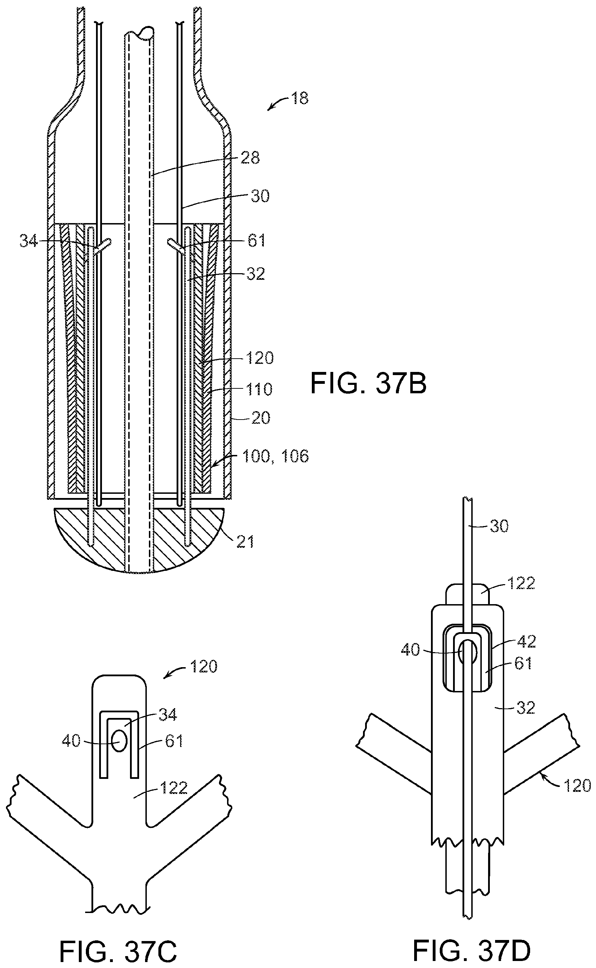

Figure 37B is an enlarged cross-sectional view of a distal end of a delivery system in accordance with an embodiment of the present technology.
Figures 37C-37D are enlarged partial side views of a valve support configured for use with the delivery system of Figure 37B in accordance with an embodiment of the present technology.

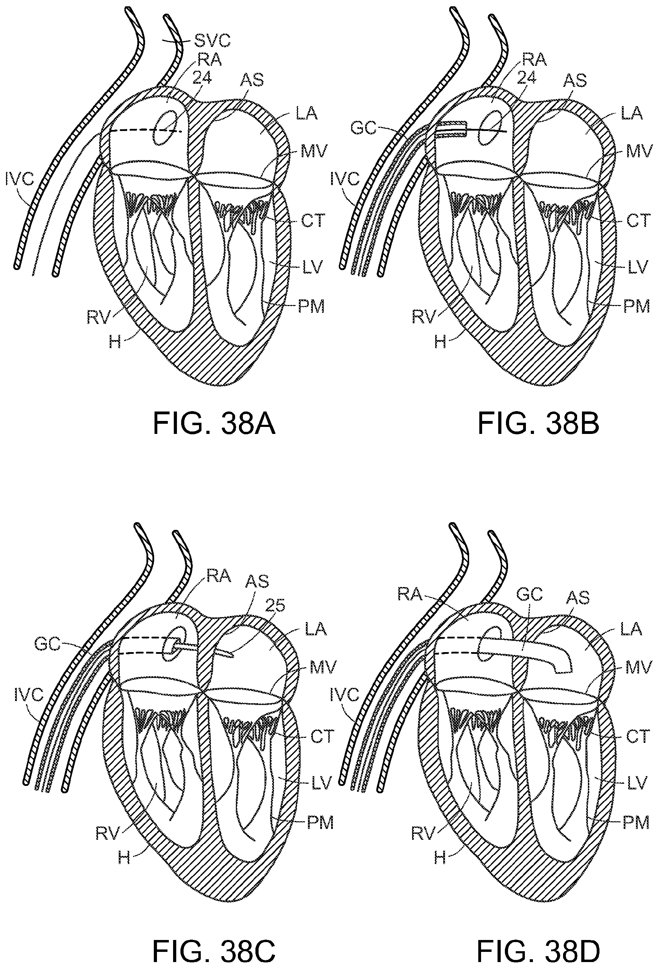

Figures 38A-38D are cross-sectional views of a heart showing an antegrade or trans-septal approach to the mitral valve in accordance with an embodiment of the present technology.

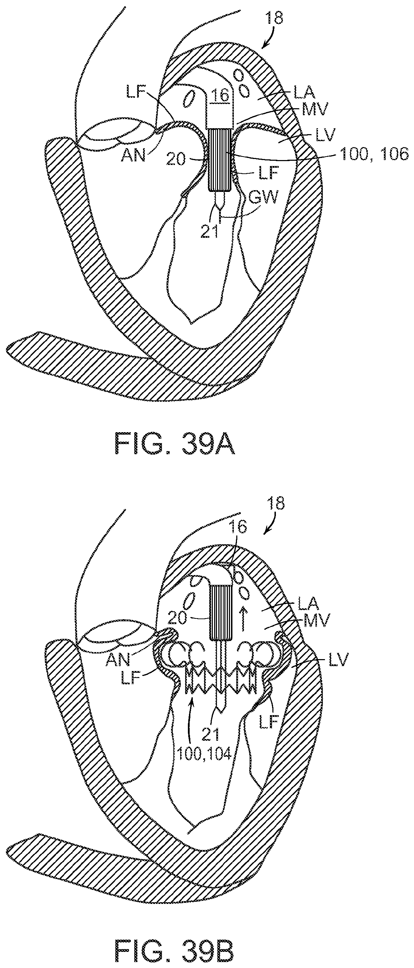

Figures 39A-39C are cross-sectional views of the heart illustrating a method of implanting a prosthetic heart valve device using a trans-septal approach in accordance with another embodiment of the present technology.

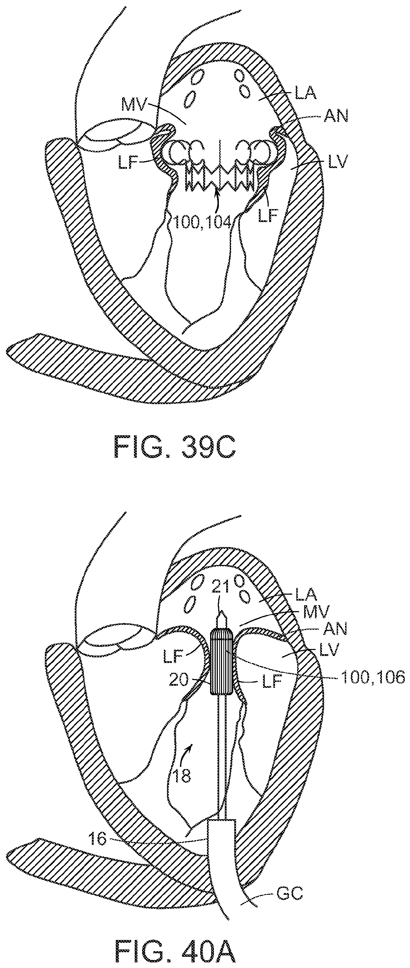

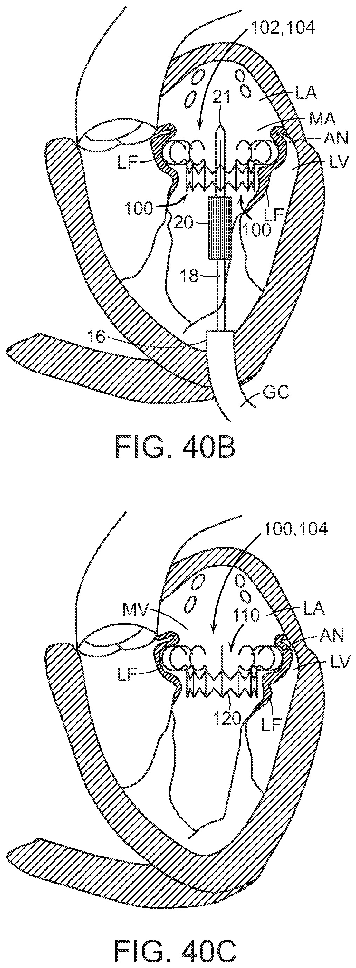

Figures 40A-40C are cross-sectional views of the heart illustrating a further embodiment of a method of implanting the prosthetic heart valve device using a trans-apical approach in accordance with aspects of the present technology.

### DETAILED DESCRIPTION

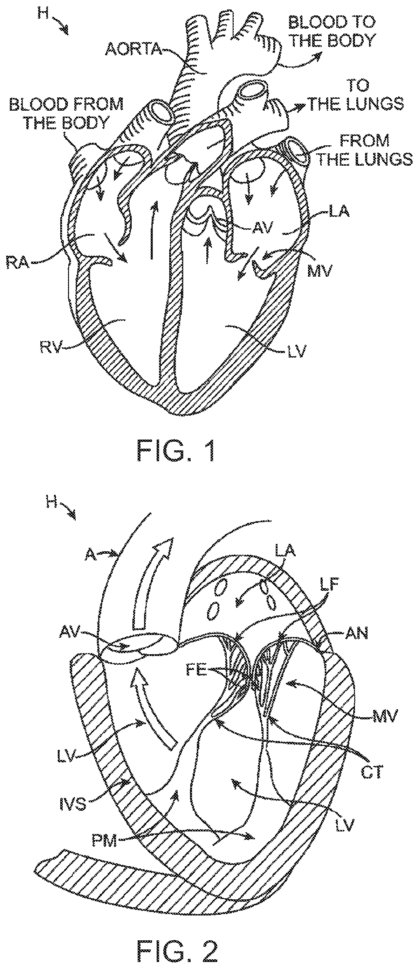

Specific details of several embodiments of the technology are described below with reference to Figures 1-40C. Although many of the embodiments are described below with respect to devices, systems, and methods for percutaneous replacement of a native mitral valve using prosthetic valve devices, other applications and other embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described below with reference to Figures 1-40C. The invention is defined by claim 1. Preferred embodiments are defined by the dependent claims.

With regard to the terms "distal" and "proximal" within this description, unless otherwise specified, the terms can reference a relative position of the portions of a prosthetic valve device and/or an associated delivery device with reference to an operator and/or a location in the vasculature or heart. For example, in referring to a delivery catheter suitable to deliver and position various prosthetic valve devices described herein, "proximal" can refer to a position closer to the operator of the device or an incision into the vasculature, and "distal" can refer to a position that is more distant from the operator of the device or further from the incision along the vasculature (e.g., the end of the catheter). With respect to a prosthetic heart valve device, the terms "proximal" and "distal" can refer to the location of portions of the device with respect to the direction of blood flow. For example, proximal can refer to an upstream position or a position of blood inflow, and distal can refer to a downstream position or a position of blood outflow. For ease of reference, throughout this disclosure identical reference numbers and/or letters are used to identify similar or analogous components or features, but the use of the same reference number does not imply that the parts should be construed to be identical. Indeed, in many examples described herein, the identically numbered parts are distinct in structure and/or function. The headings provided herein are for convenience only.

### Overview

Systems, devices and methods are provided herein for percutaneous replacement of native heart valves, such as mitral valves. Several of the details set forth below are provided to describe the following examples and methods in a manner sufficient to enable a person skilled in the relevant art to practice, make and use them. Several of the details and advantages described below, however, may not be necessary to practice certain examples and methods of the technology. Additionally, the technology may include other examples that are within the scope of the claims but are not described in detail.

Embodiments of the present technology provide apparatus to treat valves of the body, such as heart valves including the mitral valve. The apparatus enable a percutaneous approach using a catheter delivered intravascularly through a vein or artery into the heart. Additionally, the apparatus enable other less-invasive approaches including trans-apical, trans-atrial, and direct aortic delivery of a prosthetic replacement valve to a target location in the heart. The apparatus enable a prosthetic device to be anchored at a native valve location by engagement with a subannular surface of the valve annulus and/or valve leaflets. Additionally, the embodiments of the devices as described herein can be combined with many known surgeries and procedures, for example combined with known methods of accessing the valves of the heart such as the mitral valve or triscuspid valve with antegrade or retrograde approaches, and combinations thereof.

The devices described herein provide a valve replacement device that has the flexibility to adapt and conform to the variably-shaped native mitral valve anatomy while mechanically isolating the prosthetic valve from the anchoring portion of the device, which can absorb the distorting forces applied by the native anatomy. The device has the structural strength and integrity necessary to withstand the dynamic conditions of the heart over time, thus permanently anchoring a replacement valve and making it possible for the patient to resume a substantially normal life. The devices further deliver such a device in a less-invasive manner, providing a patient with a new, permanent replacement valve but also with a lower-risk procedure and a faster recovery.

The devices described herein provide a valve replacement device that has the flexibility to adapt and conform to the variably-shaped native mitral valve anatomy while simultaneously providing the structural strength and integrity necessary to withstand the dynamic conditions of the heart over time, thus permanently anchoring a replacement valve, making it possible for the patient to resume a substantially normal life. The devices further deliver such a device in a less-invasive manner, providing a patient with a new, permanent replacement valve but also with a lower-risk procedure and a faster recovery.

In accordance with various embodiments of the present technology, a device for repair or replacement of a native heart valve, wherein the native heart valve has an annulus and leaflets coupled to the annulus is disclosed. The device can include a valve support having an upstream end and a downstream end extending around a longitudinal axis, and have an outer surface and an inner surface. The valve support can have a cross-sectional shape and the inner surface can be configured to support a prosthetic valve. The device can also include an expandable retainer that is couple dot the upstream end of the valve support. The retainer can be configured to engage tissue on or downstream of the annulus. In various embodiments, the valve support is mechanically isolated from the retainer such that the cross-sectional shape of the valve support remains sufficiently stable when the retainer is deformed in a non-circular shape by engagement with the tissue.

Some embodiments of the disclosure are directed to prosthetic heart valve devices for treating a mitral valve. The device can include a valve support configured to support a valve. The device can also include a retainer coupled to the valve support and positionable at least partially along a subannular surface of a native mitral valve annulus. The retainer can also inhibit upstream migration of the device. The retainer is coupled to the valve support so as to mechanically isolate the valve support from distorting force exerted on the retainer by native anatomy.

In some embodiments, the device may comprise an atrial extension member extending from the retainer to a position at least partially upstream of the native mitral annulus. In other embodiments, the device may further comprise a plurality of arms extending radially outward from the valve support. The arms can be configured to engage native leaflets of the mitral valve, for example. Some embodiments of the device may further comprise one or more stabilizing members for engaging subannular tissue and limiting movement of the device in an upstream or downstream direction.

In a further embodiment, a prosthetic heart valve device for treating a mitral valve can include an expandable retainer configured to engage cardiac tissue at or downstream of a native mitral valve annulus. The device can also include a valve support coupled to and at least partially surrounded by the expandable retainer. The valve support can be configured to support a prosthetic valve such as either a temporary valve, or in other embodiments, a permanent valve structure. In these arrangements, the expandable retainer is configured to conform to the shape of the native mitral valve annulus while the valve support remains substantially unchanged.

In yet a further embodiment, a prosthetic heart valve device for treating a heart valve in a patient can include a valve support having a generally circular shape and configured to support a prosthetic valve, and a deformable retainer coupled to an upstream portion of the valve support. The deformable retainer can be configured to engage cardiac tissue on or below an annulus of the heart valve. The valve support can be mechanically isolated from the retainer such that deformation of the retainer does not substantially affect the generally circular shape of the valve support. The device may also include a plurality of arms coupled to a downstream portion of the valve support. The arms can be biased outward from the valve support in an unbiased configuration such that the plurality of arms can be configured to engage a native mitral leaflet.

The disclosure further provides systems for delivery of prosthetic valves and other devices using endovascular or other minimally invasive forms of access. For example, embodiments of the present technology provide a system to treat a mitral valve of a patient, in which the mitral valve has an annulus. The system comprises a device to treat the mitral valve as described herein and a catheter having a lumen configured to retain the device within the catheter.

For exemplary reasons, a method of treating a heart valve of a patient is disclosed. The mitral valve has an annulus and leaflets coupled to the annulus. The method can include implanting a device as described herein within or adjacent to the annulus. The device, in some embodiments, can include a valve support coupled to and at least partially surrounded by a deformable retainer. The deformable retainer can be coupled to an upstream end of the valve support. The deformable retainer can be disposed between the leaflets and be configured to engage tissue on or near the annulus to prevent migration of the device in an upstream direction. Further, the valve support can be mechanically isolated from the deformable retainer such that a cross-sectional shape of the valve support does not substantially change if the retainer is deformed by engagement with the tissue.

In yet a further example, a method for replacement of a native heart valve having an annulus and a plurality of leaflets is disclosed. The method can include positioning a prosthetic device as described herein between the leaflets, while the device is in a collapsed configuration. The method can also include allowing the prosthetic device to expand such that a retainer of the prosthetic device is in a subannular position in which it engages tissue on or downstream of the annulus. The retainer can have a diameter larger than a corresponding diameter of the annulus in the subannular position. The method can further include allowing a valve support to expand within the retainer, wherein the valve support is coupled to the retainer at an upstream end of the valve support. In various embodiments, the valve support can be mechanically isolated from the retainer such that deformation of the retainer when the retainer engages the tissue does not substantially deform the valve support.

The devices and methods disclosed herein can be configured for treating non-circular, asymmetrically shaped valves and bileaflet or bicuspid valves, such as the mitral valve. Many of the devices and methods disclosed herein can further provide for long-term (e.g., permanent) and reliable anchoring of the prosthetic device even in conditions where the heart or native valve may experience gradual enlargement or distortion.

### Cardiac and Mitral Valve Physiology

Figures 1 and 2 show a normal heart H. The heart comprises a left atrium that receives oxygenated blood from the lungs via the pulmonary veins PV and pumps this oxygenated blood through the mitral valve MV into the left ventricle LV. The left ventricle LV of a normal heart H in systole is illustrated in Figure 2. The left ventricle LV is contracting and blood flows outwardly through the aortic valve AV in the direction of the arrows. Back flow of blood or "regurgitation" through the mitral valve MV is prevented since the mitral valve is configured as a "check valve" which prevents back flow when pressure in the left ventricle is higher than that in the left atrium LA.

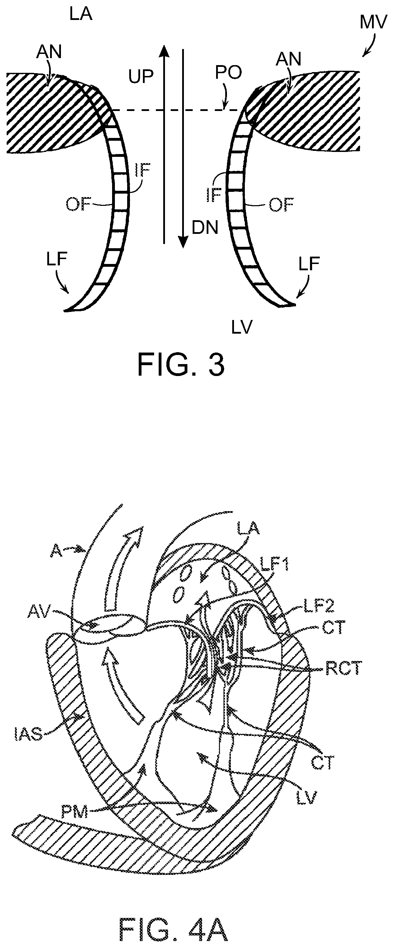

The mitral valve MV comprises a pair of leaflets having free edges FE which meet evenly, or "coapt" to close, as illustrated in Figure 2. The opposite ends of the leaflets LF are attached to the surrounding heart structure via an annular region of tissue referred to as the annulus AN. Figure 3 is a schematic cross-sectional side view of an annulus and leaflets of a mitral valve. As illustrated, the opposite ends of the leaflets LF are attached to the surrounding heart structure via a fibrous ring of dense connective tissue referred to as the annulus AN, which is distinct from both the leaflet tissue LF as well as the adjoining muscular tissue of the heart wall. The leaflets LF and annulus AN are comprised of different types of cardiac tissue having varying strength, toughness, fibrosity, and flexibility. Furthermore, the mitral valve MV may also comprise a unique region of tissue interconnecting each leaflet LF to the annulus AN, referred to herein as leaflet/annulus connecting tissue LAC (indicated by overlapping cross-hatching). In general, annular tissue AN is tougher, more fibrous, and stronger than leaflet tissue LF.

Referring to Figure 2, the free edges FE of the mitral leaflets LF are secured to the lower portions of the left ventricle LV through chordae tendineae CT (referred to hereinafter "chordae") which include a plurality of branching tendons secured over the lower surfaces of each of the valve leaflets LF. The chordae CT in turn, are attached to the papillary muscles PM, which extend upwardly from the lower wall of the left ventricle LV and interventricular septum IVS.

Referring now to Figures 4A to 4B, a number of structural defects in the heart can cause mitral valve regurgitation. Ruptured chordae RCT, as shown in Figure 4A, can cause a valve leaflet LF2 to prolapse since inadequate tension is transmitted to the leaflet via the chordae. While the other leaflet LF1 maintains a normal profile, the two valve leaflets do not properly meet and leakage from the left ventricle LV into the left atrium LA will occur, as shown by the arrow.

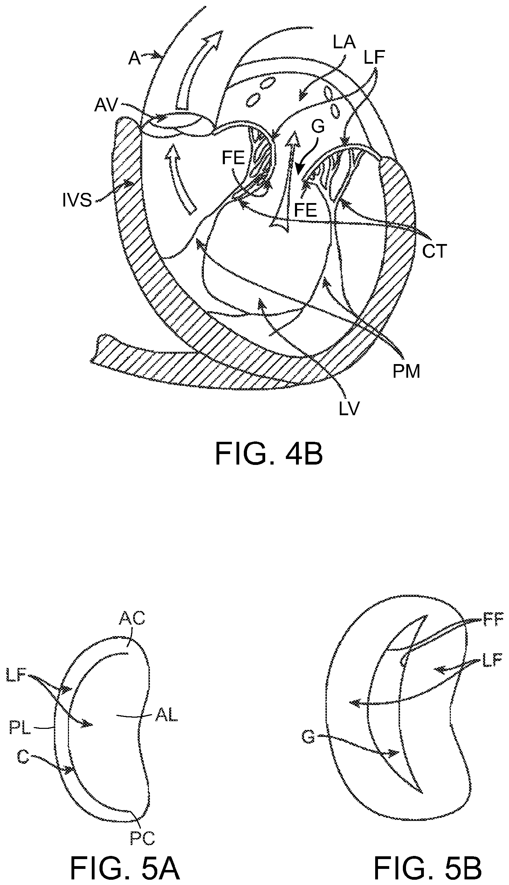

Regurgitation also occurs in the patients suffering from cardiomyopathy where the heart is dilated and the increased size prevents the valve leaflets LF from meeting properly, as shown in Figure 4B. The enlargement of the heart causes the mitral annulus to become enlarged, making it impossible for the free edges FE to meet during systole. The free edges of the anterior and posterior leaflets normally meet along a line of coaptation C as shown in Figure 5A, but a significant gap G can be left in patients suffering from cardiomyopathy, as shown in Figure 5B.

Mitral valve regurgitation can also occur in patients who have suffered ischemic heart disease where the functioning of the papillary muscles PM is impaired, as illustrated in Figure 4A. As the left ventricle LV contracts during systole, the papillary muscles PM do not contract sufficiently to effect proper closure. One or both of the leaflets LF1 and LF2 then prolapse. Leakage again occurs from the left ventricle LV to the left atrium LA.

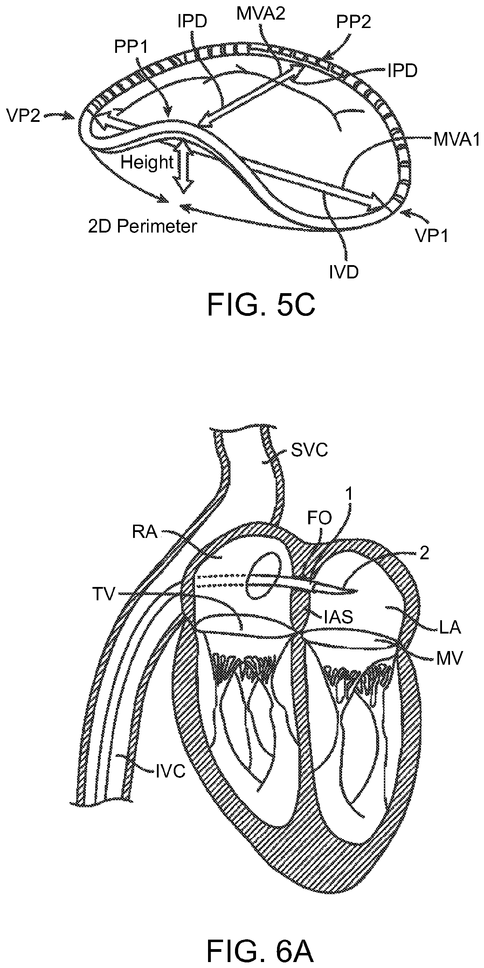

Figures 5A-5C further illustrate the shape and relative sizes of the leaflets L of the mitral valve. Referring to Figure 5C, it may be seen that the overall valve has a generally "D"- or kidney-like shape, with a long axis MVA1 and a short axis MVA2. In healthy humans the long axis MVA1 is typically within a range from about 33.3 mm to about 42.5 mm in length (37.9 +/- 4.6 mm), and the short axis MVA2 is within a range from about 26.9 to about 38.1 mm in length (32.5 +/- 5.6 mm). However, with patients having decreased cardiac function these values can be larger, for example MVA1 can be within a range from about 45 mm to 55 mm and MVA2 can be within a range from about 35 mm to about 40 mm. The line of coaptation C is curved or C-shaped, thereby defining a relatively large anterior leaflet AL and substantially smaller posterior leaflet PL (Figure 5A). Both leaflets appear generally crescent-shaped from the superior or atrial side, with the anterior leaflet AL being substantially wider in the middle of the valve than the posterior leaflet. As illustrated in Figure 5A, at the opposing ends of the line of coaptation C the leaflets join together at corners called the anterolateral commissure AC and posteromedial commissure PC, respectively.

Figure 5C shows the shape and dimensions of the annulus of the mitral valve. The annulus is an annular area around the circumference of the valve comprised of fibrous tissue which is thicker and tougher than that of the leaflets LF and distinct from the muscular tissue of the ventricular and atrial walls. The annulus may comprise a saddle-like shape with a first peak portion PP1 and a second peak portion PP2 located along an interpeak axis IPD, and a first valley portion VP1 and a second valley portion VP2 located along an intervalley axis IVD. The first and second peak portion PP1 and PP2 are higher in elevation relative to a plane containing the nadirs of the two valley portions VP1, VP2, typically being about 8-19 mm higher in humans, thus giving the valve an overall saddle-like shape. The distance between the first and second peak portions PP1, PP2, referred to as interpeak span IPD, is substantially shorter than the intervalley span IVD, the distance between first and second valley portions VP1, VP2.

A person of ordinary skill in the art will recognize that the dimensions and physiology of the patient may vary among patients, and although some patients may comprise differing physiology, the teachings as described herein can be adapted for use by many patients having various conditions, dimensions and shapes of the mitral valve. For example, work in relation to embodiments suggests that some patients may have a long dimension across the annulus and a short dimension across the annulus without well-defined peak and valley portions, and the methods and device as described herein can be configured accordingly.

### Access to the Mitral Valve

Access to the mitral valve or other atrioventricular valve can be accomplished through the patient's vasculature in a percutaneous manner. By percutaneous it is meant that a location of the vasculature remote from the heart is accessed through the skin, typically using a surgical cut down procedure or a minimally invasive procedure, such as using needle access through, for example, the Seldinger technique. The ability to percutaneously access the remote vasculature is well-known and described in the patent and medical literature. Depending on the point of vascular access, the approach to the mitral valve may be antegrade and may rely on entry into the left atrium by crossing the inter-atrial septum. Alternatively, approach to the mitral valve can be retrograde where the left ventricle is entered through the aortic valve. Once percutaneous access is achieved, the interventional tools and supporting catheter(s) may be advanced to the heart intravascularly and positioned adjacent the target cardiac valve in a variety of manners, as described herein.

Using a trans-septal approach, access is obtained via the inferior vena cava IVC or superior vena cava SVC, through the right atrium RA, across the inter-atrial septum IAS and into the left atrium LA above the mitral valve MV.

As shown in Figure 6A, a catheter 1 having a needle 2 may be advanced from the inferior vena cava IVC into the right atrium RA. Once the catheter 1 reaches the anterior side of the inter-atrial septum IAS, the needle 2 may be advanced so that it penetrates through the septum, for example at the fossa ovalis FO or the foramen ovate into the left atrium LA. At this point, a guidewire may be exchanged for the needle 2 and the catheter 1 withdrawn.

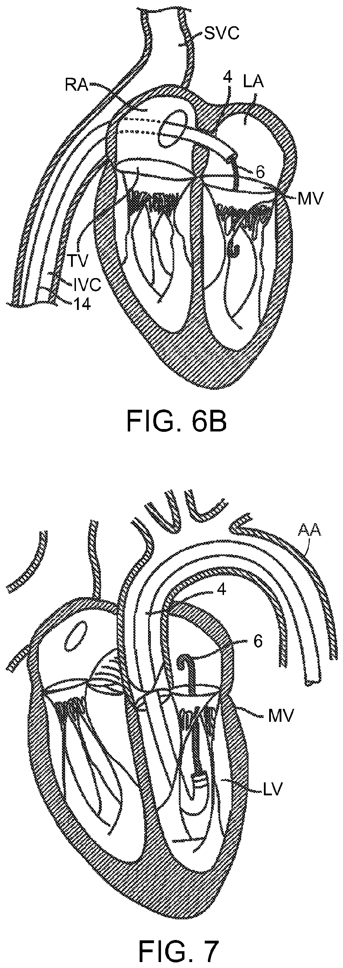

As shown in Figure 6B, access through the inter-atrial septum IAS may usually be maintained by the placement of a guide catheter 4, typically over a guidewire 6 which has been placed as described above. The guide catheter 4 affords subsequent access to permit introduction of the device to replace the mitral valve, as described in more detail herein.

In an alternative antegrade approach (not shown), surgical access may be obtained through an intercostal incision, preferably without removing ribs, and a small puncture or incision may be made in the left atrial wall. A guide catheter may then be placed through this puncture or incision directly into the left atrium, sealed by a purse-string suture.

The antegrade or trans-septal approach to the mitral valve, as described above, can be advantageous in many respects. For example, the use of the antegrade approach will usually allow for more precise and effective centering and stabilization of the guide catheter and/or prosthetic valve device. Precise positioning facilitates accuracy in the placement of the prosthetic valve device. The antegrade approach may also reduce the risk of damaging the subvalvular device during catheter and interventional tool introduction and manipulation. Additionally, the antegrade approach may decrease risks associated with crossing the aortic valve as in retrograde approaches. This can be particularly relevant to patients with prosthetic aortic valves, which cannot be crossed at all or without substantial risk of damage.

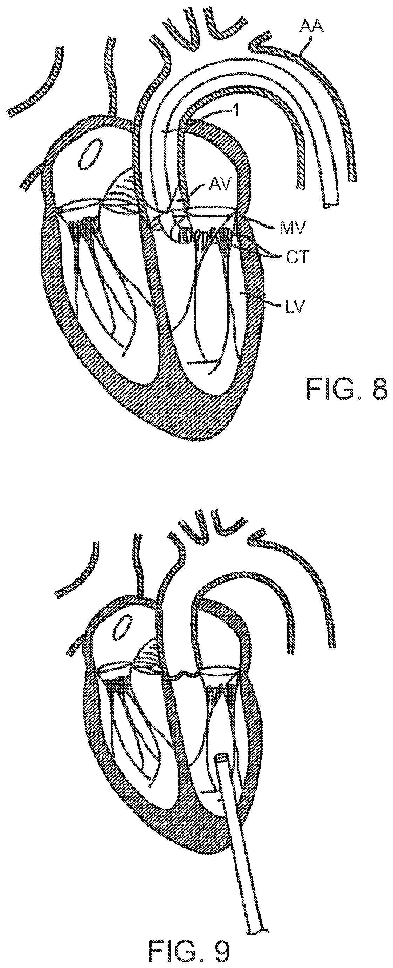

An example of a retrograde approach to the mitral valve is illustrated in Figures 7 and 8. The mitral valve MV may be accessed by an approach from the aortic arch AA, across the aortic valve AV, and into the left ventricle LV below the mitral valve MV. The aortic arch AA may be accessed through a conventional femoral artery access route, as well as through more direct approaches via the brachial artery, axillary artery, radial artery, or carotid artery. Such access may be achieved with the use of a guidewire 6. Once in place, a guide catheter 4 may be tracked over the guidewire 6. Alternatively, a surgical approach may be taken through an incision in the chest, preferably intercostally without removing ribs, and placing a guide catheter through a puncture in the aorta itself. The guide catheter 4 affords subsequent access to permit placement of the prosthetic valve device, as described in more detail herein.

In some specific instances, a retrograde arterial approach to the mitral valve may be choosen due to certain advantages. For example, use of the retrograde approach can eliminate the need for a trans-septal puncture. The retrograde approach is also more commonly used by cardiologists and thus has the advantage of familiarity.

An additional approach to the mitral valve is via trans-apical puncture, as shown in Figure 9. In this approach, access to the heart is gained via thoracic incision, which can be a conventional open thoracotomy or sternotomy, or a smaller intercostal or sub-xyphoid incision or puncture. An access cannula is then placed through a puncture, sealed by a purse-string suture, in the wall of the left ventricle at or near the apex of the heart. The catheters and prosthetic devices of the invention may then be introduced into the left ventricle through this access cannula.

The trans-apical approach has the feature of providing a shorter, straighter, and more direct path to the mitral or aortic valve. Further, because it does not involve intravascular access, the trans-apical procedure can be performed by surgeons who may not have the necessary training in interventional cardiology to perform the catheterizations required in other percutaneous approaches.

The prosthetic treatment device may be specifically designed for the approach or interchangeable among approaches. A person of ordinary skill in the art can identify an appropriate approach for an individual patient and design the treatment apparatus for the identified approach in accordance with embodiments described herein.

Orientation and steering of the prosthetic valve device can be combined with many known catheters, tools and devices. Such orientation may be accomplished by gross steering of the device to the desired location and then refined steering of the device components to achieve a desired result.

Gross steering may be accomplished by a number of methods. A steerable guidewire may be used to introduce a guide catheter and the prosthetic treatment device into the proper position. The guide catheter may be introduced, for example, using a surgical cut down or Seldinger access to the femoral artery in the patient's groin. After placing a guidewire, the guide catheter may be introduced over the guidewire to the desired position. Alternatively, a shorter and differently shaped guide catheter could be introduced through the other routes described above.

A guide catheter may be pre-shaped to provide a desired orientation relative to the mitral valve. For access via the trans-septal approach, the guide catheter may have a curved, angled or other suitable shape at its tip to orient the distal end toward the mitral valve from the location of the septal puncture through which the guide catheter extends. For the retrograde approach, as shown in Figures 7 and 8, guide catheter 4 may have a pre-shaped J-tip which is configured so that it turns toward the mitral valve MV after it is placed over the aortic arch AA and through the aortic valve AV. As shown in Figure 7, the guide catheter 4 may be configured to extend down into the left ventricle LV and to assume a J-shaped configuration so that the orientation of an interventional tool or catheter is more closely aligned with the axis of the mitral valve MV. In either case, a pre-shaped guide catheter may be configured to be straightened for endovascular delivery by means of a stylet or stiff guidewire which is passed through a lumen of the guide catheter. The guide catheter might also have pull-wires or other means to adjust its shape for more fine steering adjustment.

### Selected Embodiments of Prosthetic Heart Valve Devices and Methods

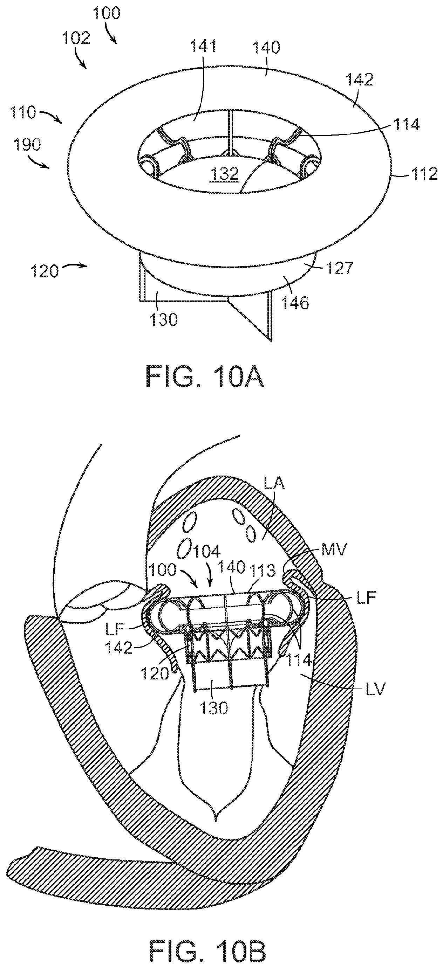

Embodiments of the present technology as described herein can be used to treat one or more of the valves of the heart as described herein, and in particular embodiments, can be used for treatment of the mitral valve. Examples of prosthetic heart valve devices, system components and associated methods in accordance with embodiments of the present technology are described in this section with reference to Figures 10A-40C. It will be appreciated that specific elements, substructures, advantages, uses, and/or other features of the embodiments described with reference to Figures 10A-40C can be suitably interchanged, substituted or otherwise configured with one another in accordance with additional embodiments of the present technology. Furthermore, suitable elements of the embodiments described with reference to Figures 10A-40C can be used as stand-alone and/or self-contained devices.

Systems, devices and methods are provided herein for percutaneous implantation of prosthetic heart valves in a heart of a patient. In some embodiments, methods and devices are presented for the treatment of valve disease by minimally invasive implantation of artificial replacement heart valves. In one embodiment, the artificial replacement valve can be a prosthetic valve device suitable for implantation and replacement of a mitral valve between the left atrium and left ventricle in the heart of a patient. In another embodiment, the prosthetic valve device can be suitable for implantation and replacement of another valve (e.g., a bicuspid or tricuspid valve) in the heart of the patient. Figure 10A shows an isometric view of a prosthetic heart valve device 100 in an expanded configuration 102 in accordance with an embodiment of the present technology, and Figure 10B is a schematic illustration of a cross-sectional view of a heart depicting the left atrium, left ventricle, and native mitral valve of the heart. Figure 10B also shows an embodiment of the expandable prosthetic valve device 100 implanted in the native mitral valve region of the heart.

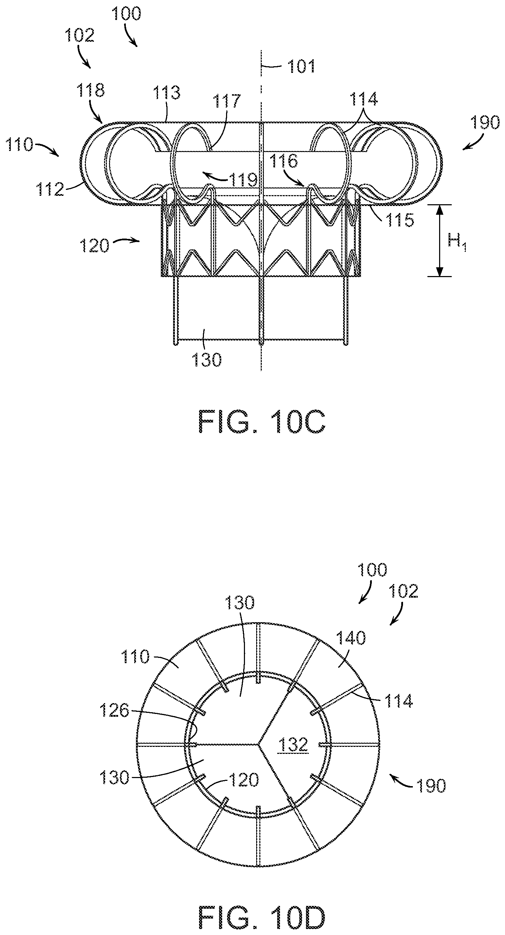

As shown in Figure 10A, the device 100 can include an expandable retainer 110 at least partially surrounding and coupled to an inner valve support 120. The device 100 can further include a prosthetic valve 130 coupled to, mounted within, or otherwise carried by the valve support 120. Figures 10C-10D are side and top views, respectively, of the prosthetic heart valve device 100 in accordance with the present technology. Referring to Figure 10A, the device 100 can also include one or more sealing members 140 that can extend around an inner surface 141 or outer surface 142 (as shown) of the retainer 110 and/or around an interior surface 126 (shown in Figure 10D) or exterior surface 127 (shown in Figure 10A) of the valve support 120 to prevent paravalvular (e.g., paraprosthetic) leaks between the device 100 and the native tissue and/or between the retainer 110 and the valve support 120.

The prosthetic heart valve device 100 can be movable between a delivery configuration (not shown), an expanded configuration 102 (Figure 10A), and a deployed configuration 104 (Figure 10B). In the delivery configuration, the prosthetic heart valve device 100 has a low profile suitable for delivery through small-diameter guide catheters positioned in the heart via the trans-septal, retrograde, or trans-apical approaches described herein. In some embodiments, the delivery configuration of the prosthetic heart valve device 100 will preferably have an outer diameter no larger than about 8-10 mm for trans-septal approaches, about 8-10 mm for retrograde approaches, or about 8-12 mm for trans-apical approaches to the mitral valve MV. As used herein, "expanded configuration" refers to the configuration of the device when allowed to freely expand to an unrestrained size without the presence of constraining or distorting forces. "Deployed configuration," as used herein, refers to the device once expanded at the native valve site and subject to the constraining and distorting forces exerted by the native anatomy.

Referring back to Figure 3, "subannular," as used herein, refers to a portion of the mitral valve MV that lies on or downstream DN of the plane PO of the native orifice. As used herein, the plane PO of the native valve orifice is a plane generally perpendicular to the direction of blood flow through the valve and which contains either or both the major axis MVA1 or the minor axis MVA2 (Figure 5C). Thus, a subannular surface of the mitral valve MV is a tissue surface lying on the ventricular side of the plane PO, and preferably one that faces generally downstream, toward the left ventricle LV. The subannular surface may be disposed on the annulus AN itself or the ventricular wall behind the native leaflets LF, or it may comprise a surface of the native leaflets LF, either inward-facing IF or outward-facing OF, which lies below the plane PO. The subannular surface or subannular tissue may thus comprise the annulus AN itself, the native leaflets LF, leaflet/annulus connective tissue, the ventricular wall or combinations thereof.

In operation, the prosthetic heart valve device 100 can be intravascularly delivered to a desired location in the heart, such as an intracardiac location near the mitral valve MV, while in the delivery (e.g., collapsed) configuration within a delivery catheter (not shown). Referring to Figure 10B, the device 100 can be advanced to a position within or downstream of the native annulus AN where the device 100 can be released from the delivery catheter to enlarge toward the expanded configuration 102 (Figure 10A). The device 100 will engage the native tissue at the desired location, which will deform or otherwise alter the shape of the device 100 into the deployed configuration 104 (Figure 10B). Once released from the catheter, the device 100 can be positioned such that at least a portion of the expandable retainer 110 engages a subannular surface of the native valve so as to resist systolic forces and prevent upstream migration of the device 100 (Figure 10B). In the embodiment illustrated in Figure 10B, an upstream perimeter 113 of the retainer 110 engages the inward-facing surfaces IF (Figure 3) of the native leaflets LF, which are pushed outwardly and folded under the native annulus AN. The leaflets LF engage a ventricular side of the annulus AN and are prevented from being pushed further in the upstream direction, thus maintaining the retainer 110 below the plane of the native valve annulus. In some embodiments, however, some portions of the retainer 110 may extend above the annulus AN, with at least some portions of the retainer 110 engaging tissue in a subannular location to prevent migration of the device 100 toward the left atrium LA. As shown in Figure 10B, the leaflets LF can lie in apposition against the outer surface 142 of the retainer 110 forming a blood-tight seal with the sealing member 140.

In accordance with aspects of the present technology, the expandable retainer 110, while in a deployed configuration 104, conforms to the irregularly-shaped mitral annulus AN, effectively sealing the device 100 against the native annulus AN to anchor the device and to prevent paravalvular leaks. As described further herein, the retainer 110 mechanically isolates the valve support 120 from distorting forces present in the heart such that the retainer 110 may adapt and/or conform to native forces while the valve support 120 maintains its structural integrity. Accordingly, the retainer 110 can be sufficiently flexible and resilient and/or coupled to the valve support 120 in such a manner as to mechanically isolate the valve support 120 from the forces exerted upon the retainer 110 by the native anatomy. Alternatively, or in addition to the above features, the valve support 120 may be more rigid and/or have greater radial strength than the radial strength of the retainer 110 so as to maintain its cylindrical or other desired shape and to ensure proper opening and closing of the prosthetic valve 130 housed within the valve support structure 120. In some embodiments, the valve support 120 has a radial strength of at least 100%, or in other embodiments at least 200%, and in further embodiments at least 300%, greater than a radial strength of the retainer 110. In one embodiment, the valve support 120 can have a radial strength of approximately 10N to about 12N. Thus, if deformed from its unbiased shape by exerting a radially compressive force against its circumference, the valve support 120 can exhibit a hoop force which is about 2 to about 20 times greater for a given degree of deformation than will be exhibited by the retainer 110..

The retainer 110 comprises a flexible, upstream portion of the device 100 and is implanted such that at least a portion of the retainer 110 engages tissue at or near the native mitral annulus. The retainer 110 can be a generally outward oriented portion of the device 100, as shown in Figure 10C. In one embodiment, the retainer 110 forms a donut-shaped flange 190 having an arcuate outer surface 142 for engaging tissue and an inner lumen defining a passage for blood to flow through the valve support 120. In another example, the outer surface 142 can have other shapes, such as linear, triangular, an irregular shape, etc. In some embodiments, the retainer 110 can include a plurality of circumferentially positioned, resiliently deformable and flexible ribs 114 which are coupled at their downstream ends 116 to the valve support 120. Once deployed, at least a portion of the upstream region 118 of the flexible ribs 114 can expand outward from the valve support 120 to engage a surface at or near the native valve (e.g., mitral valve).

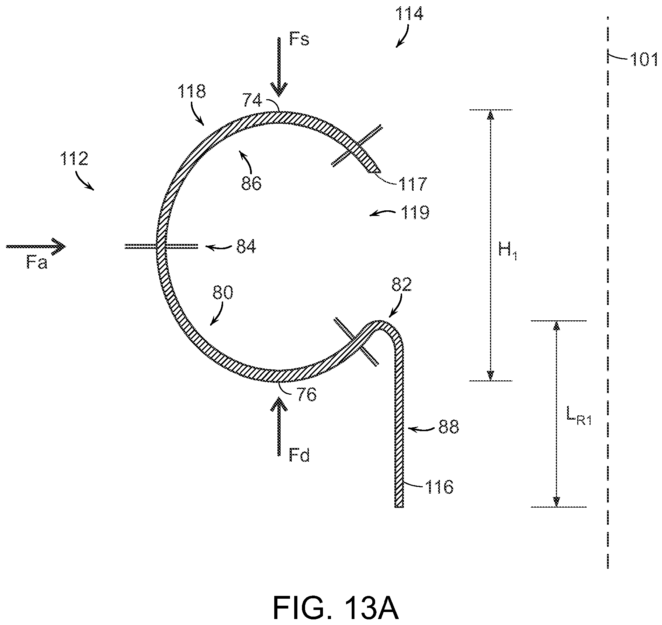

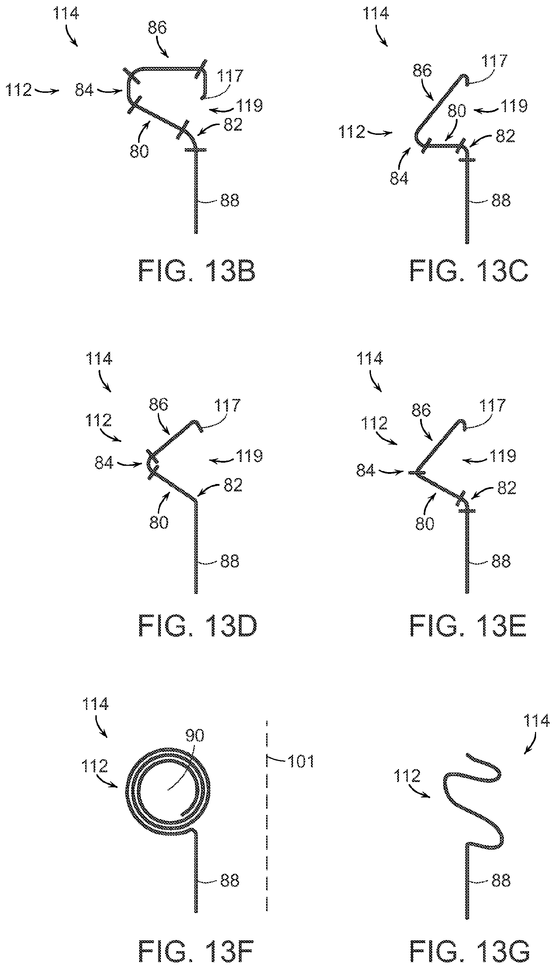

Additionally, Figures 10A-10D also illustrate that the flexible ribs 114, in one embodiment, can have a general C-shape configuration with tips 117 of the flexible ribs 114 and opening 119 of the C-shape configuration oriented toward a longitudinal axis 101 of the device 100. As shown in Figures 10A-10D, the each individual flexible rib 114 can be independent or otherwise unconnected to any other (e.g., adjacent) flexible rib 114 of the retainer 110. However, in some embodiments, not shown, the retainer 110 can have circumferential connectors connecting one or more flexible ribs 114 of the retainer 110. In some embodiments, the flexible ribs 114 may be divided along their length into multiple, separated segments (shown below with respect to Figures 13A-13G). The plurality of flexible ribs 114 can be formed from a deformable material or from a resilient or shape memory material (e.g., nitinol). In other embodiments, the retainer 110 can comprise a mesh or woven construction in addition to or in place of the flexible ribs 114. For example, the retainer 110 could include a plurality of flexible wires or filaments arranged in a diamond pattern or other configuration. In a particular example, the retainer 110 can be formed of a pre-shaped nitinol tube having, for example, a wall thickness of approximately 0.010 inches to about 0.130 inches.

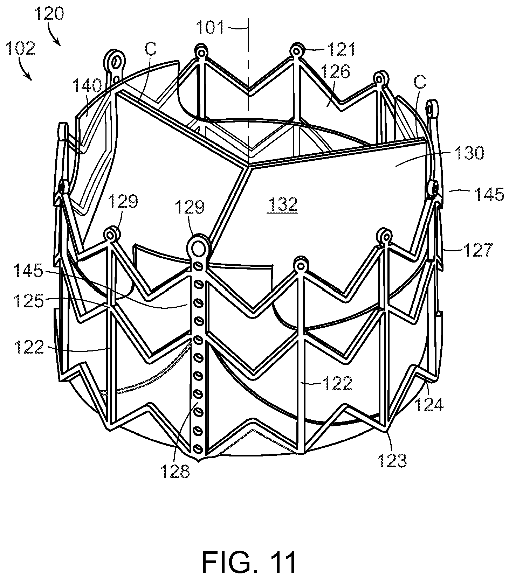

Figure 11 shows an embodiment of the valve support 120 that can be used in the various embodiments of the prosthetic heart valve device 100 shown in Figures 10A-10D. Figure 11 is an isometric view of the valve support 120 shown in an expanded configuration 102 in accordance with the present technology. Referring to Figures 10A-10D and 11 together, several embodiments of the valve support 120 can be generally cylindrical having an upstream end 121 and a downstream end 123 formed around the longitudinal axis 101 with a circular, oval, elliptical, kidney-shaped, D-shaped, or other suitable cross-sectional shape configured to support a tricuspid or other prosthetic valve 130. In some embodiments, the valve support 120 includes a plurality of posts 122 connected circumferentially by a plurality of struts 124. The posts 122 and struts 124 can be arranged in a variety of geometrical patterns that can expand and provide sufficient resilience and column strength for maintaining the integrity of the prosthetic valve 130. For example, the plurality of posts 122 can extend longitudinally across multiple rows of struts 124 to provide column strength to the valve support 120. However, in other embodiments, the valve support 120 can include a metallic, polymeric, or fabric mesh or woven construction.

Generally, the plurality of posts 122 can extend along an axial direction generally parallel to the longitudinal axis 101 and the struts 124 can extend circumferentially around and transverse to the longitudinal axis 101. The posts 122 can extend an entire longitudinal height H₁ (shown in Figure 10C) of the valve support 120 and in one embodiment the height H₁ can be approximately 14 mm to about 17 mm. Referring to Figure 11, the struts 124 can form a series of rings around the longitudinal axis 101, wherein each ring has a circumferentially expandable geometry. In the example shown in Figure 11, the struts 124 are formed in a series of zig-zags to form a chevron configuration. Alternative expandable geometries can include sinusoidal patterns, diamond configurations, closed cells, open cells, or other expandable configurations. The plurality of struts 124 can attach to the plurality of posts 122 so as to define a plurality of nodes 125 where the struts and posts intersect. The plurality of struts 124 and the plurality of posts 122 can be formed from a deformable material or from a resilient or shape memory material (e.g., nitinol).

As shown in Figure 11, the valve support 120 has the interior surface 126 and the exterior surface 127, and the valve support 120 is configured to receive the prosthetic valve 130 within an interior lumen of the valve support 120 to inhibit retrograde blood flow (e.g., blood flow from the left ventricle into the left atrium). Accordingly, the valve support 120 can provide a scaffold to which prosthetic valve tissue can be secured and provide a scaffold that has sufficient axial rigidity to maintain a longitudinal position of the prosthetic valve 130 relative to the retainer 110. The valve support 120 can further provide such a scaffold having radial rigidity to maintain circularity (or other desired cross-sectional shape) to ensure that leaflets 132 of the prosthetic valve 130 coapt or otherwise seal when the device 100 is subject to external radial pressure. In one embodiment, the valve support 120 can have a support region 145 along the longitudinal axis 101 that is configured to attach to the prosthetic valve, or in other embodiments, be aligned with the coaptation portion of the leaflets 132 (shown in Figure 11).

The valve 130 may comprise a temporary or permanent valve adapted to block blood flow in the upstream direction and allow blood flow in the downstream direction through the valve support 120. The valve 130 may also be a replacement valve configured to be disposed in the valve support 120 after the device 100 is implanted at the native mitral valve. The leaflets 132 may be formed of various flexible and impermeable materials including PTFE, Dacron^{®}, pyrolytic carbon, or other biocompatible materials or biologic tissue such as pericardial tissue or xenograft valve tissue such as porcine heart tissue or bovine pericardium. Other aspects of valve 130 are described further below.

The interior surface 126 within the lumen of the valve support 120 can be covered at least partially by an impermeable sealing member 140 to prevent blood flow from inside the valve support 120 to the outside of the valve support 120, where it could leak around the exterior of the valve support 120. In another embodiment, the sealing member 140 may be affixed to the exterior surface 127 of the valve support 120 and, in either embodiment, may be integrally formed with or attached directly to valve 130. In an additional embodiment, the sealing member 140 can be applied on at least portions of both the interior surface 126 and the exterior surface 127 of the valve support 120.

In accordance with aspects of the present technology and as shown in Figure 11, the prosthetic valve 130 can be sutured, riveted, glued, bonded, or otherwise fastened to posts 122 or commissural attachment structures 128, which are configured to align with valve commissures C. The posts 122 or commissural attachment structures 128 can include eyelets 129, loops, or other features formed thereon to facilitate attachment of sutures or other fastening means to facilitate attachment of the prosthetic valve 130. In one embodiment, as shown in Figure 11, the attachment structures 128 can be integrated into the structural frame of the valve support 120 such that the attachment structures 128 are distributed around the circumference of the valve support 120 and function as posts 122. In other embodiments, not shown, the attachment structures 128 can be attachment pads formed on parts of the posts 122 (e.g., along an upper end of the posts 122) or can be separate structures that can be coupled to posts 122, struts 124 or other components along the interior surface 126 of the valve support 120. The prosthetic valve 130 may also be attached to the sealing member 140, which can be a sleeve attached to the interior surface 126 of the valve support 120.

Once attached, the prosthetic valve 130 can be suitable to collapse or compress with the device 100 for loading into a delivery catheter (not shown). In one embodiment, the prosthetic valve 130 has a tri-leaflet configuration, although various alternative valve configurations may be used, such as a bi-leaflet configuration. The design of the prosthetic valve 130, such as the selection of tri-leaflet vs. bi-leaflet configurations, can be used to determine the suitable shape of the valve support 120. For example, for a tri-leaflet valve, the valve support 120 can have a circular cross-section, while for a bi-leaflet valve, alternative cross-sectional shapes are possible such as oval or D-shaped cross-sections. In particular examples, the valve support can have a circular cross-sectional diameter of approximately 25 mm to about 30 mm, such as 27 mm.

In some arrangements, the valve support 120 can have a permanent prosthetic valve pre-mounted therein, or the valve support 120 may be configured to receive a separate catheter-delivered valve following implantation of the device 100 at the native mitral valve. In arrangements where a permanent or replacement valve is desirable, the valve support 120 can further include a temporary valve pre-mounted within the interior lumen. If a period of time between placement of the device 100 and further implantation of the permanent prosthetic valve is desirable, a temporary valve sewn into or otherwise secured within the valve support 120 can assure regulation of blood flow in the interim. For example, temporary valves may be used for a period of about 15 minutes to several hours or up to a several days. Permanent or replacement prosthetic valves may be implanted within a temporary valve or may be implanted after the temporary valve has been removed. Examples of pre-assembled, percutaneous prosthetic valves include, e.g., the CoreValve ReValving^{®} System from Medtronic/Corevalve Inc. (Irvine, CA, USA), or the Edwards-Sapien^{®} valve from Edwards Lifesciences (Irvine, CA, USA). If adapted to receive a separate catheter-delivered valve, the valve support 120 may have features within its interior lumen or on its upper or lower ends to engage and retain the catheter-delivered valve therein, such as inwardly extending ridges, bumps, prongs, or flaps. Additional details and embodiments regarding the structure, delivery and attachment of prosthetic valves, temporary valves and replacement valves suitable for use with the prosthetic heart valve devices disclosed herein can be found in International PCT Patent Application No. PCT/US2012/043636, entitled "PROSTHETIC HEART VALVE DEVICES AND ASSOCIATED SYSTEMS AND METHODS," filed June 21, 2012, the entire contents of which are incorporated herein by reference.

In some embodiments, a downstream portion 111 of the retainer 110 can be coupled to or near the upstream end 121 of the valve support 120 and extend outward and in an upstream direction from the valve support 120 in a manner that does not unduly influence the shape of the valve support 120. Accordingly, in some embodiments, the retainer 110 can be configured to engage and deform to the shape of the native tissue on or under the annulus while a cross-sectional shape of the valve support 120 remains sufficiently stable or substantially undeformed. For example, the valve support 120 (e.g., at least at the upstream end 121) can be spaced longitudinally downstream from at least a tissue engaging portion 112 of the retainer 110 such that if the retainer 110 is deformed inwardly, the cross-sectional shape of the valve support 120, which remains positioned downstream of the tissue engaging portion 112 of the retainer 110, remains substantially undeformed. As used herein, "substantially undeformed" can refer to situations in which the valve support 120 is not engaged or deformed, or can refer to scenarios in which the valve support 120 can deform slightly but the prosthetic valve 130 remains intact and competent (e.g., the leaflets 132 coapt sufficiently to prevent retrograde blood flow). In such arrangements, leaflets 132 of the prosthetic valve 130 can close sufficiently even when the device 100 is under systolic pressures or forces from the pumping action of the heart.

As illustrated in Figures 10A-10D, the retainer 110 can be coupled to or near the upstream end 121 of the valve support 110 the valve support 120 in such that the valve support 120 and valve 130 reside within the left ventricle. Alternatively, the retainer 110 can be coupled to the valve support 120 anywhere along a length of the valve support 120 such that the valve support 120 and valve 130 can reside within the annulus or above the annulus of the native heart valve. The valve support 120 and retainer 110 may be coupled by a variety of methods known in the art, e.g., suturing, soldering, welding, bonding, staples, rivets or other fasteners, mechanical interlocking, friction, interference fit, or any combination thereof.

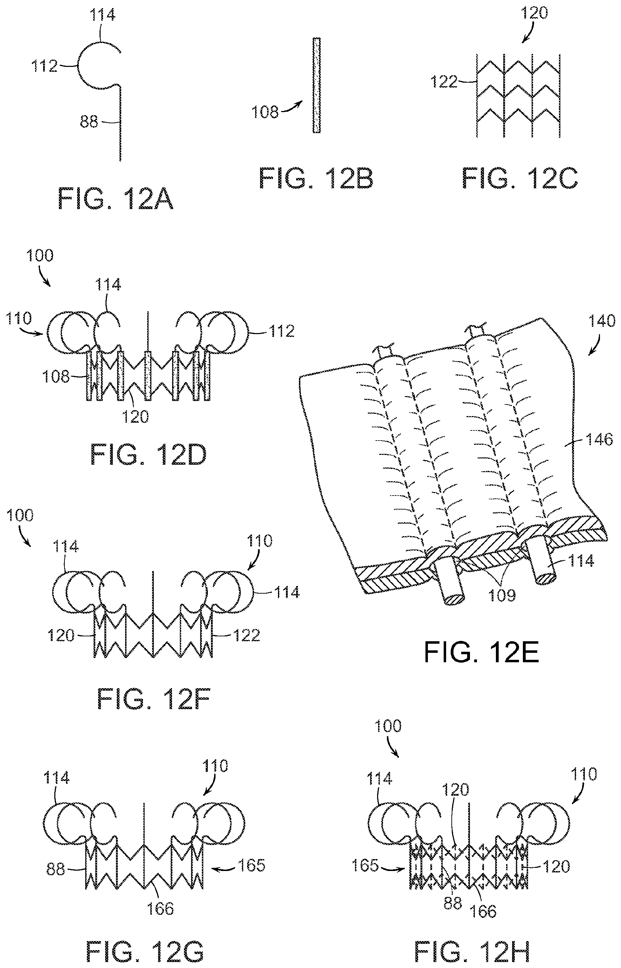

Figures 12A-12H are side views of additional mechanisms of coupling the valve support 120 to the retainer 110 that can allow mechanical isolation of the valve support 120 from the retainer 110 in accordance with additional embodiments of the present technology. Referring to Figures 12A-12D, the flexible ribs 114 can include rib posts 88 (Figure 12A) that can be coupled to valve support posts 122 (Figure 12C) using individual hypotubes 108 (shown in Figure 12B). For example, as shown in Figure 12D, the rib post 88 may be aligned with the individual valve support posts 122 and the hypotube 112 may be slipped over both the valve support posts 122 and the rib posts 88. The hypotubes 108 can be crimped or otherwise adhered to valve support posts 122 and the rib posts 88 such that the flexible ribs 114 are connected to and aligned with valve support posts 122 in a manner that allows the tissue engaging portions 112 to extend outward and in an upstream direction from the valve support 120.

If the retainer 110 and the valve support are separate structures, a sealing member 140 or other overlaying structure may be attached to both the retainer 110 and the valve support 120 to interconnect the two structures. For example, the valve support 120 can be covered by a sealing member 140, such as a sleeve 146 that includes a plurality of longitudinal pockets 109 formed (e.g., by suturing or bonding two layers of sleeve fabric together) or otherwise incorporated circumferentially around the sleeve 146. As shown in Figure 12E, each individual rib 114 can be constrained within the pockets 109 formed in the sleeve 146, and the sleeve can be coupled to an interior or exterior surface 126, 127 of the valve support (Figure 11). In other embodiments, the valve support 120 and the retainer 110 can be integrally formed with one another. For example, the flexible ribs 114 can be formed integrally with the posts 122 of the valve support 120 (shown in Figures 10C and 12F.

In a further embodiment shown in Figures 12G-12H, the retainer 110 may include a retainer frame 165, separate from the frame of the valve support 120 (Figure 12G). The retainer frame 165, in one embodiment, may include rib posts 88 connected circumferentially by deformable and/or flexible connectors 166, and can be configured to receive or partially surround the valve support 120 (Figure 12H). In one arrangement, the retainer frame 165 can be delivered by catheter and deployed at a target site in the native heart valve and the valve support 120 can be delivered separately following deployment and implantation of the retainer frame 165. In another arrangement, the retainer frame 165 can be configured to receive or be coupled to the support frame 120 prior to delivery of the device 100 to the target site.

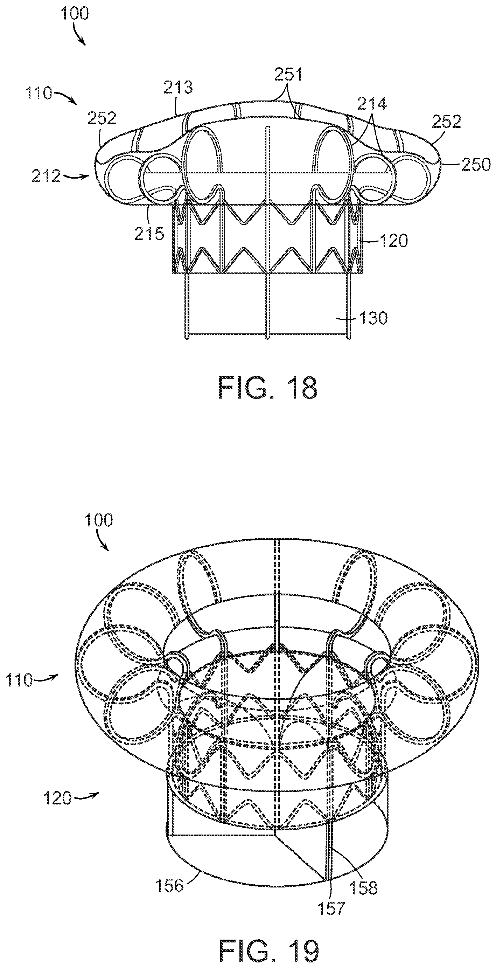

Referring back to Figures 10A-10D, the flexible ribs 114 can be less rigid than the posts 122 and/or struts 124 of the valve support 120, allowing greater flexibility in the retainer 110 and/or more stability to the shape and position of the valve support 120. In some embodiments, the flexibility of the retainer 110 can allow the retainer 110 to absorb distorting forces as well as allow the device 100 to conform to the irregular, non-circular shape of the native annulus (while leaving the valve support 120 substantially unaffected), encouraging tissue ingrowth and creating a seal to prevent leaks between the device 100 and the native tissue. In addition, the flexible ribs 114 can be configured to press radially outward against the native valve, ventricular and/or aortic structures so as to anchor the device 100 in a desired position, as well as maintain an upstream deployed circumference 150' larger than that of the native annulus such that subannular positioning effectively prevents upstream migration of the device 100 (described further below in Figure 18C). Furthermore, the flexible ribs 114 can have sufficient resilience and column strength (e.g., axial stiffness) to prevent longitudinal collapse of the retainer 110 and/or the device 100 and to resist movement of the device 100 in an upstream direction.

In accordance with embodiments of the present technology, the valve 130 and valve support 120 are effectively mechanically isolated from the distorting forces exerted on the retainer 110 by the native tissue, e.g., radially compressive forces exerted by the native annulus and/or leaflets, longitudinal diastolic and systolic forces, hoop stress, etc. For example, deformation of the retainer 110 by the native tissue can change a cross-section of the retainer 110 (e.g., to a non-circular or non-symmetrical cross-section), while the valve support 120 may be substantially undeformed. In one embodiment, at least a portion of the valve support 120 can be deformed by the radially compressive forces, for example, where the retainer 110 is coupled to the valve support 120 (e.g., the downstream end 123). However, the upstream end 121 of the valve support 120 and/or the valve support region 145 (Figure 11) is mechanically isolated from the retainer 110 and the compressive forces such that at least the valve support region 145 can be substantially undeformed. Thus the valve support 120, and at least the valve support region 145, can maintain a circular or other desirable cross-section so that the valve remains stable and/or competent. The flexibility of the ribs 114 can contribute to the absorption of the distorting forces, and also aid in mechanically isolating the valve support 120 and valve 130 from the retainer 110 and from the native anatomy.

As shown in Figure 10C, the retainer 110 is comprised of a series of circumferentially positioned flexible ribs 114 which are coupled or otherwise integrated at their downstream ends 116 to the valve support 120. Unlike valve support posts 122, the flexible ribs 114 may not be circumferentially connected by struts which can allow for greater movement, flexing, bending, rotating and/or deformation of the individual ribs 114 and the retainer 110 as a whole. In certain embodiments in which the retainer 110 did include circumferential struts or supports (not shown) for retaining or connecting the ribs 114, the struts may be more flexible than the struts 124 utilized in the valve support 120.

Figures 13A-13G are partial side views of a variety of flexible rib configurations in accordance with additional embodiments of the present technology. Referring to Figure 13A, the ribs 114, in one embodiment, can generally have an arcuate or C-shaped tissue engaging portion 112 and include the rib post 88 at the downstream end 116 of the rib 114. In some embodiments, the rib post 88 can be generally linear and have a suitable length L_{R1} for extending the retainer 110 a desirable distance upstream from a connection (not shown) to the valve support 120. In some embodiments, the rib post 88 can be generally parallel to the longitudinal axis 101 of the device 100 and/or valve support 120 (shown in Figure 10C). Following the general curvature of the C-shaped tissue engaging portion 112 shown in Figure 13A, a first segment 80 of the tissue engaging portion 112 can extend radially outward from the rib post 88 beginning at a first transition 82. The first transition 82 can be a curved or U-shaped section as shown to orient the first segment 82 outward from the rib post 88. The first segment 80 can be arcuate or generally curved in an outward and upstream direction to reach a second transition 84. A second segment 86 of the tissue engaging portion 112 can be arcuate or generally curved and extend (e.g., relative to the rib post 88) from the second transition 84 in an upstream and inward direction. The second segment 86 can also curve slightly downstream at the rib tip 117. The opening 119 of the C-shaped tissue engaging portion 112 of the rib 114 is created in the space between the first transition 82 and the rib tip 117.

Additional embodiments of rib shapes are shown in Figures 13B-13G. For example, rib segments, such as the first and second segments 80, 86, can be generally linear (Figures 13B-13E). Other embodiments of tissue engaging portion 112 can have transitions 82 and 84 with less curvature or greater curvature. For example, the first transition segment 82 may comprise a curved section with a distinct inflection point (shown in Figures 13A and 13C-13E) or a short continuous segment with a constant radial curve (Figure 12B). The tissue engaging portion 112 can also include additional transitions and/or segments to form desirable rib shapes, such as generally square-shaped (Figure 13B), or generally triangular-shaped (Figures 13C-13E) tissue engaging portions 112. Similar to the embodiment of the tissue engaging portion 112 shown in Figure 13A, the tissue engaging portion 112 shown in Figures 13B-13E have the openings 119 which can face inward toward a retainer interior (shown in Figures 10A-10D); however, one of ordinary skill in the art will recognize that the ribs 114 can be oriented in a different direction, such as having the opening 119 facing outward with respect the longitudinal axis 101 of the device 100 (not shown). Additional embodiments of the tissue engaging portion 112 can be formed without openings 119.

In other embodiments, the tissue engaging portion 112 may take on other unique geometries. As shown in Figure 12F, the tissue engaging portion 112 may coil or extend around an axis 90 transverse to the longitudinal axis 101. Or, as shown in Figure 12G, the tissue engaging portion 112 may have multiple segments extending radially outward and/or multiple segments extending radially inward with respect to the rib post 88 in an irregular or in a patterned configuration.

Referring back to Figure 13A, the tissue engaging portion 112 can have a height H₁ between an upper surface 74 and a lower surface 76. Accordingly, in addition to the shape of the tissue engaging portion 112, the overall height H₁ of the tissue engaging portion 112 can be selected to accommodate the anatomy at the desired target location of the heart valve.

Referring again to Figure 13A, the tissue engaging portion 112 of the rib 114 can be configured to absorb, translate and/or mitigate distorting forces present with the heart during, for example, systole and diastole. The shape of the tissue engaging portion 112 can be selected to accommodate forces, such as radially compressive forces, e.g., exerted by the native annulus and/or leaflets Fa, longitudinal diastolic Fd and systolic Fs forces, hoop stress, etc. Absorption of the distorting forces can serve to mechanically isolate the retainer 110 from the valve support 120. In accordance with the present technology, the ribs 114 may flex, bend, rotate or twist under the distorting forces while the valve support 120 substantially maintains its rigidity and/or original shape (e.g., a generally circular shape). In a particular example, the device 100 can include a tricuspid valve 130 retained within a generally circular valve support 120 (Figures 10A-11). When deployed and operational, the cross-sectional shape of the valve support 120 can remain sufficiently stable when the retainer 110 is deformed in a non-circular shape by engagement with the tissue such that the valve 130 remains competent.

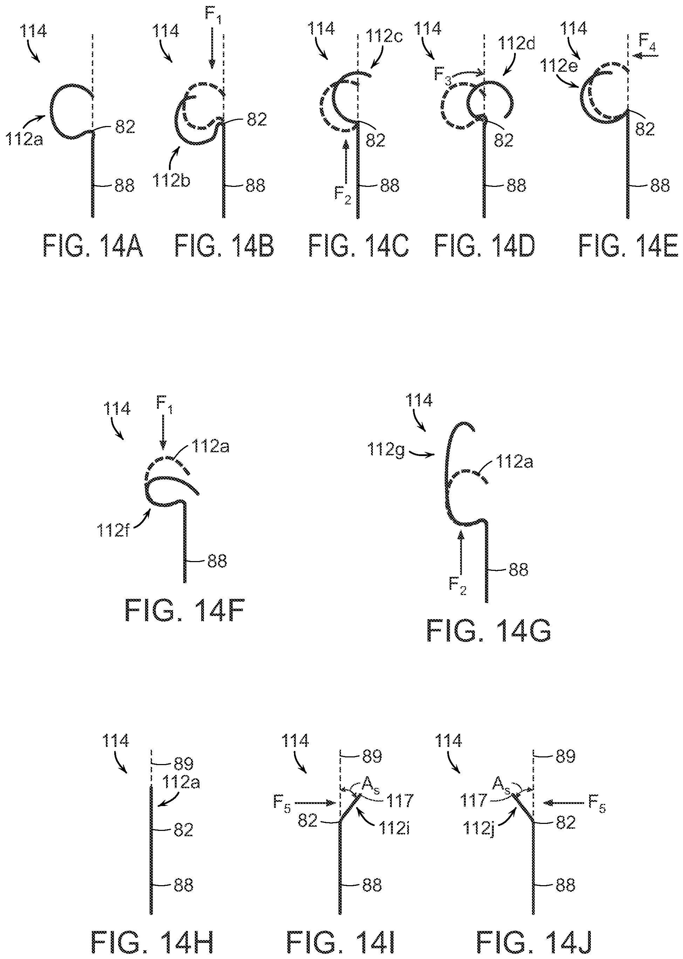

Figures 14A-14J are side views of various flexible ribs 114 flexing in response to a distorting force F in accordance with further embodiments of the present technology. The degree of flexibility of individual ribs 114 (and thus the retainer 110) may be consistent among all ribs 114 of a retainer 110, or, alternatively, some ribs 114 may be more flexible than other ribs 114 within the same retainer 110. Likewise, a degree of flexibility of individual ribs 114 may be consistent throughout an entire length of the rib 114 or curvature of the tissue engaging portion 112, or the degree of flexibility can vary along the length and/or curvature of each rib 114.

As shown Figures 14A-14J, the tissue engaging portions 112 of the ribs 114 may flex relative to the rib post 88 in response to varying distorting forces F that can be applied by the surrounding tissue during or after implantation of the device 100. From a static position (Figure 14A), the tissue engaging portion 112a may flex downward to a position 112b (Figure 14B) or upward to a position 112c (Figure 14C) in response to a downward force F₁ or an upward force F₂, respectively. Similarly, the tissue engaging portion 112a may flex inward to a position 112d (Figure 14D) or outward to a position 112e (Figure 14E) in response to a laterally directed inward force F₃ or a laterally directed outward force F₄, respectively. As shown in Figures 14A-14E, the tissue engaging portion 112a may flex and/or rotate inwardly/outwardly in response to the laterally directed forces F₃, F₄, or upward/downward in response to the generally vertically directed forces F₁, F₂ without altering the general shape of the tissue engaging portion 112. In one embodiment, the position of the tissue engaging portion 112 can occur by flex or rotation around the first transition 82 (Figures 14A-14E).

In other arrangements, the rib 114 can be configured to alter the shape of the tissue engaging portion 112a in response to forces, such as to the shape/position 112f in response to the downward force F₁ (Figure 14F) and to the shape/position 112g in response to the upward force F₂ (Figure 14G). Alteration of the shape and/or position of the tissue engaging portion 112, as shown in Figures 14F-14G, may occur by flexing, rotating and/or deformation around segments 80, 86 and/or transitions 82, 84, for example. As shown in Figures 14H-14J, the tissue engaging portion 112a (Figure 14H) may also flex and/or rotate laterally (e.g., to positions 112i or 112j) in response to a laterally-directed force F₅, by bending at transition 82, for example, at unique and variable splay angles A_{S} off a midline 89 such that the rib tips 117 may be splayed away from each other.

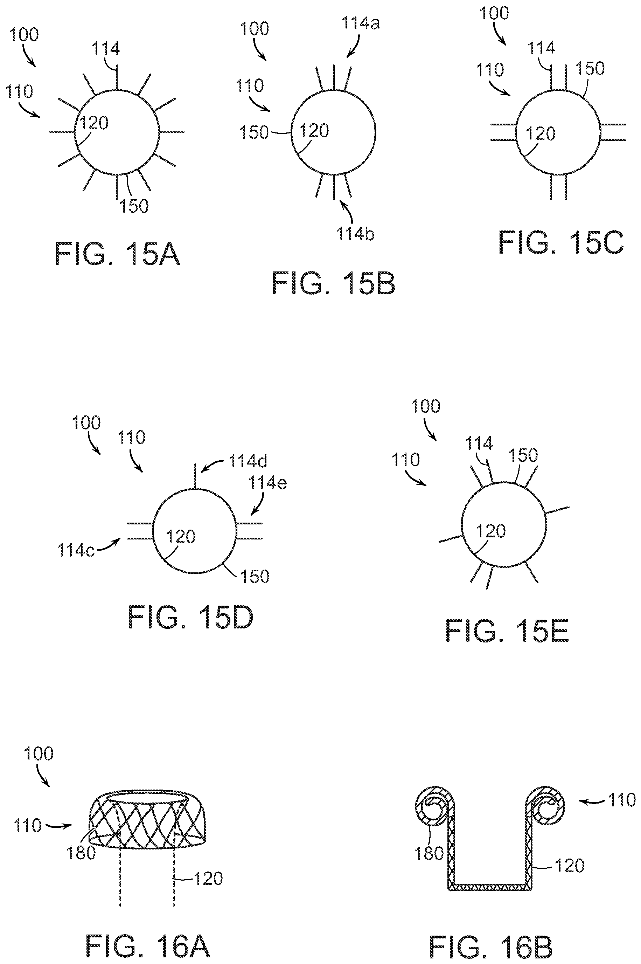

In addition to having a variety of shapes and variations in flexibility, individual ribs 114 can also be placed in a variety of positions around a circumference 150 of the retainer 110. Figures 15A-15E are schematic top views of the prosthetic heart valve device 100 showing a variety of rib configurations in accordance with further embodiments of the present technology. Figure 15A shows and embodiment of the device 100 having a plurality of ribs 114 symmetrically and evenly spaced around the circumference 150 of the retainer 110. In some embodiments, the device 100 can include a first plurality of ribs 114a and second plurality of ribs 114b (Figure 15B). In some embodiments, the first plurality of ribs 114a can have a characteristic different than the second plurality of ribs 114b. Various characteristics could include size of the rib, rib shape, rib stiffness and the number of ribs 114 within a given area of the retainer 110. As shown in Figures 15C and 15D, the retainer 110 can include multiple groups of ribs 114 spaced symmetrically (Figure 15C) or asymmetrically (Figure 15D) around the circumference 150 of the retainer 110. Referring to Figure 15C, the groups of ribs 114c and 114e may include different numbers of ribs 114 than in other groups (e.g., 114d). In other embodiments, the ribs 114 can be unevenly spaced around the circumference 150 of the retainer 110 (Figures 15E). The retainer 110 can include, in one embodiment, between approximately 2 ribs to about 30 ribs, and in another embodiment, between approximately 6 ribs to about 20 ribs.

Figures 16A-16B are schematic side and cross-sectional views of the prosthetic heart valve device 100 showing additional embodiments of the retainer 110 in accordance with the present technology. In some embodiments, the retainer 110 can be formed from a self-expanding mesh 180 or weave of material formed from a deformable material or a resilient or shape memory material (e.g., nitinol) that can evert (Figure 16A) or that can roll (Figure 16B) to form the retainer 110. In other embodiments, the retainer 110 can comprise the self-expanding mesh or woven construction in addition to the flexible ribs 114. In one embodiment, the self-expanding mesh 180 could include a plurality of flexible wires or filaments arranged in a diamond pattern (Figure 16A) or other configuration. In a particular example, the retainer 110 can be formed of a pre-shaped nitinol tube having, for example, a wall thickness of approximately 0.010 inches to about 0.130 inches.

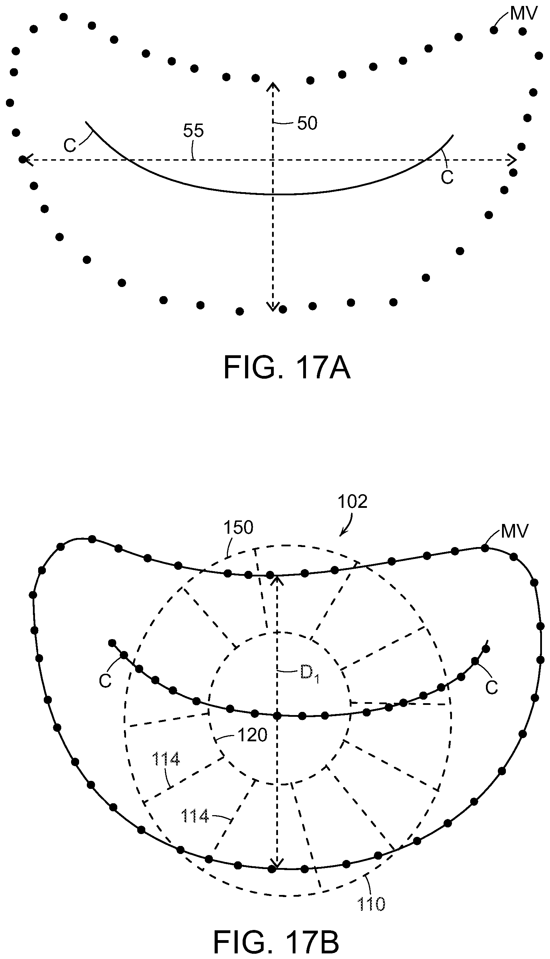

The flexible characteristics of the individual ribs 114 can allow for the flexibility and conformability of the retainer 110 to engage and seal the device 100 against uneven and uniquely-shaped native tissue. Additionally, the flexibility can assist in creating a seal between the device 100 and the surrounding anatomy. Figure 17A is a schematic top view of a native mitral valve MV illustrating the minor axis 50 and major axis 55, and Figures 17B-17C are schematic top views of an retainer 110 in an expanded configuration 102 and in a deployed configuration 104, respectively, overlaying the schematic of the native mitral valve MV in accordance with an embodiment of the present technology.

Referring to Figure 17B, the retainer 110 can have an outer circumference 150 with a diameter D₁ that is greater than the minor axis 50 (Figure 17A) of the native annulus, and usually less than the major axis 55 of the annulus, when the retainer 110 is in an expanded configuration 102 (shown as dashed lines). In other embodiments, the retainer 110 may have a diameter D₁ at least as large as the distance between the native commissures C, and may be as large as or even larger than the major axis 55 of the native annulus. In some embodiments, the outer circumference 150 of the retainer 110 has the diameter D₁ which is approximately 1.2 to 1.5 times the diameter (not shown) of the valve support 120 (or the prosthetic valve 130), and can be as large as 2.5 times the diameter of the valve support 120 (or the prosthetic valve 130). While conventional valves must be manufactured in multiple sizes to treat diseased valves of various sizes, the valve support 120 and the prosthetic valve 130, in accordance with aspects of the present technology, may be manufactured in just a single diameter to fit a multitude of native valve sizes. For example, the valve support 120 and the prosthetic valve 130 do not need to engage and fit the native anatomy precisely. In a specific example, the valve support 120 may have a diameter (not shown) in the range of about 25mm to about 32 mm for adult human patients. Also in accordance with aspects of the present technology, the retainer 110 may be provided in multiple diameters or having a variable size circumference 150 to fit various native valve sizes, and may range in diameter from about 28 mm to about 80 mm, or in other embodiments, greater than 80 mm.

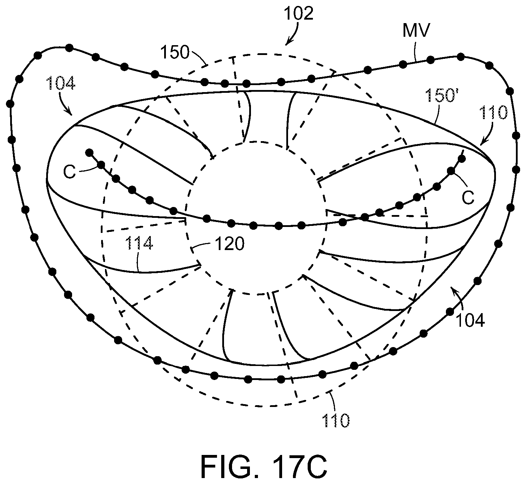

The top view of the retainer 110 shown in Figure 17C illustrates how flexibility and/or deformation of one or more flexible ribs 114 and/or rib segments allows the retainer 110 to distort relative to the expanded configuration 102, as shown by the dashed lines, into a deployed configuration 104, as shown by the bolded lines. As shown in Figure 17C, the retainer 110, when deployed or implanted at or under the mitral valve annulus, can conform to the highly variable native mitral valve tissue shape MV, as shown in the dotted lines. The ribs 114 can bend, twist, and stretch such that the overall shape of the retainer 110 has a deployed (e.g., a generally more oval or D-shaped, or other irregular shape) configuration 104 instead of a fully expanded configuration 102. Referring to Figures 17B-17C together, the retainer 110 covers the mitral valve commissures C in the deployed configuration 104, whereas the commissures C would be left unsealed or exposed in the more circular expanded configuration 102, potentially allowing paravalvular leaks. The retainer 110 could also be pre-shaped to be in a generally oval or D-shape, or other shape, when in an unbiased condition.

In many embodiments, the retainer 110 can have sufficient flexibility such that the retainer 110 conforms to the native mitral annulus when in the deployed configuration 104 (Figure 17C), however, the retainer 110 can be configured to remain biased towards its expanded configuration 102 (e.g., Figures 10A and 17B) such that, when in the deployed configuration 104, the retainer 110 pushes radially outwards against the native annulus, leaflets, and/or ventricular walls just below the annulus. In some arrangements, the radial force generated by the biased retainer shape may be sufficient to deform the native anatomy such that the minor axis 50 (Figure 17A) of the native valve is increased slightly, and/or the shape of the annulus is otherwise altered. Such radial force can enhance anchoring of the device 100 to resist movement toward the atrium when the valve 130 is closed during ventricular systole as well as movement toward the ventricle when the valve 130 is open. Furthermore, the resulting compression fit between the retainer 110 and leaflets and/or ventricular walls or other structures helps create a long-term bond between the tissue and the device 100 by encouraging tissue ingrowth and encapsulation.

Figure 18 is a side view of a prosthetic heart valve device 100 shown in an expanded configuration 102 in accordance with a further embodiment of the present technology. The device 100 can include features generally similar to the features of the prosthetic heart valve device 100 described above with reference Figures 10A-17C. For example, the device 100 includes the valve support 120 and the prosthetic valve 130 housed within an interior lumen of the valve support 120. However, in the embodiment shown in Figure 18, the device 100 includes a retainer 210 having an oval or D-shaped upstream perimeter 213 and a plurality of elevations around a circumference 250 of the retainer 210 such that the retainer 210 is suitable for engaging and conforming with tissue in the subannular region of the mitral valve.

Similar to the retainer 110 of device 100 (Figure 10A), the tissue engaging portion 212 of the retainer 210 can be a generally outward oriented portion of the device 100. As shown in Figure 18, the retainer 110 can include of a series of circumferentially positioned, resiliently deformable and flexible ribs 214. In other embodiments, the retainer 210 can include flexible wires or filaments arranged in a diamond pattern or configuration (not shown). The flexible ribs 214 can, in some embodiments, provide column strength sufficient to inhibit movement of the device 100 relative the annulus under the force of systolic blood pressure against the valve 130 mounted in the valve support 120.

In some embodiments, the upstream perimeter 213 of the retainer 210 does not lie in a single plane. For example, the ribs 214 can have variable lengths and/or be off-set from each other at variable angles such that a distance (e.g., elevation) between a downstream perimeter 215 and the upstream perimeter 213 can vary around the circumference 250. For example, the upstream perimeter 213 can form a rim having a plurality of peaks 251 and valleys 252 for adapting to the shape of the native mitral valve (see Figure 5C). As used herein, "peaks" and "valleys" refers to portions of the upstream perimeter 213 having an undulating shape formed by changes in elevation with respect to the downstream perimeter 215. In some embodiments, the peak portions of the upstream perimeter 213 are about 2 to about 20 mm, or more preferably about 5 mm to about 15 mm, higher (further upstream) than the valley portions relative to a reference plane perpendicular to the direction of blood flow through the valve.

In one embodiment, the upstream perimeter 213 of the retainer 210 can have two peaks 251 that are separated by two valleys 252. In some embodiments, a first peak can have a different shape or elevation than that of a second peak. In other embodiments, the shape of a valley 252 can be different than a shape of an inverted peak 251. Accordingly, the peaks 251 and valleys 252 can be asymmetrically positioned and shaped around the circumference 250 of the retainer 210. In various arrangements, the valleys 252 can be configured for positioning along commissural regions of the native annulus, and the peaks 251 can be configured for positioning along leaflet regions of the native annulus. In one embodiment, the peaks 251 can have apices configured to be positioned near midpoint regions of the leaflets.

Although the retainer 210 is deformable in response to distorting forces exerted by the native anatomy, the valve support 120 can have sufficient rigidity to maintain a circular or other original cross-sectional shape, thus ensuring proper functioning of the prosthetic valve leaflets 132 when opening and closing. Such mechanical isolation from the retainer 210 may be achieved by the valve support 120 having sufficient rigidity to resist deformation while retainer 210 is deformed, and by selecting a location and means for coupling the valve support 120 to the retainer 210 so as to mitigate the transmission of forces through the retainer 210 to the valve support 120 or the prosthetic valve 130 contained therein. For example, the valve support 120 may be coupled to the retainer 210 only at the upstream end 121 of the valve support 120, and the retainer 110 can further extend away from the valve support in an outward and upstream direction. Thus, forces exerted on the retainer 210 by the annulus or subannular tissue can be absorbed by the flexible ribs 214 of the retainer 210 to mitigate transmission of such forces to the valve support 120.

### Additional Components and Features Suitable for Use with the Prosthetic Heart Valve Devices

Additional components and features that are suitable for use with the prosthetic heart valve devices (e.g., devices 100 described above) are described herein. It will be recognized by one of ordinary skill in the art that while certain components and features are described with respect to a particular device (e.g., device 100), the components and features can also be suitable for use with or incorporated with other devices as described further herein.

As discussed above with respect to Figure 10A, some embodiments of the prosthetic heart valve device 100 can include a sealing member 140 that extends around portions of the retainer 110 and/or the valve support 120. For example, the embodiment illustrated in Figure 10A has a sealing member 140 around the outer surface 142 of the retainer 110 and around an exterior surface 127 of the valve support 120 to prevent paravalvular leaks both between the device 100 and the anatomy but also through components of the device 100. Additionally, the sealing member 140 can be configured to promote in-growth of tissue for facilitating implantation of the device 100 in the native heart valve. In one embodiment, the sealing member can be a sleeve 146 (Figure 10A) which can include an impermeable sealing material that is cylindrical and configured to fit within or over various frame or skeleton structures of the device 100 as further described below.

In Figure 10A, the sleeve 146 is on the exterior surface 127 of the valve support 120; however, in other embodiments, the sleeve 146 or other sealing member 140 can be disposed on the interior surface 126 of the valve support 120. While Figure 10A illustrates an embodiment of the device 100 in which the sleeve 146 is disposed on the outer surface 142 of the retainer 110, one of ordinary skill will recognize other configurations where the sleeve 146 can be disposed on the inner surface 141 of the retainer 110.

One of ordinary skill in the art will recognize that the sealing members 140, such as the sleeves 146, can fully cover the surfaces 126, 127, 141 and 142 or in other embodiments, at least partially cover the surfaces 126, 127, 141 and 142 of the retainer 110 and the valve support 120, respectively. Any combination of sealing members 140 is contemplated. Additionally, the sealing member 140 can comprise a single continuous sheet of fluid impervious material (e.g., for covering a surface 141, 142 of the retainer 110 and a surface 126, 127 of the valve support 120), which could create a seal between the retainer 110 and the valve support 120. In various embodiments, the sealing member 140, such as the sleeve 146, can comprise a fabric or other flexible and biocompatible material such as Dacron^{®}, ePTFE, bovine pericardium, or other suitable flexible material to integrate with tissue and minimize paravalvular leaks. In other embodiments, the sealing member 140 can include a polymer, thermoplastic polymer, polyester, Gore-tex^{®}, a synthetic fiber, a natural fiber or polyethylene terephthalate (PET). The valve 130 may also be attached to the sealing member 140 or integrally formed with the sealing member 140.

The prosthetic heart valve device 100 can also include additional support features for maintaining a desired shape and/or rigidity of the valve support 120 or the retainer 110. Figure 19 is an isometric view of the prosthetic heart valve device 100 having a connecting ring 156 in accordance with an embodiment of the present technology. As shown in Figure 19, the connecting ring 156 can be coupled to plurality of commissure posts 158 integrated and/or coupled to the valve support 120. As shown in Figure 19, the connecting ring 156 can be coupled to the downstream ends 157 of the commissure posts 158; however, the connecting ring 156 may also be coupled to another portion of the commissure posts 158 or the valve support 120. The connecting ring 156 can have a variety of symmetrical or non-symmetrical geometrical cross-sections and can provide support for the commissure posts 158 to keep the posts from bending or deforming.

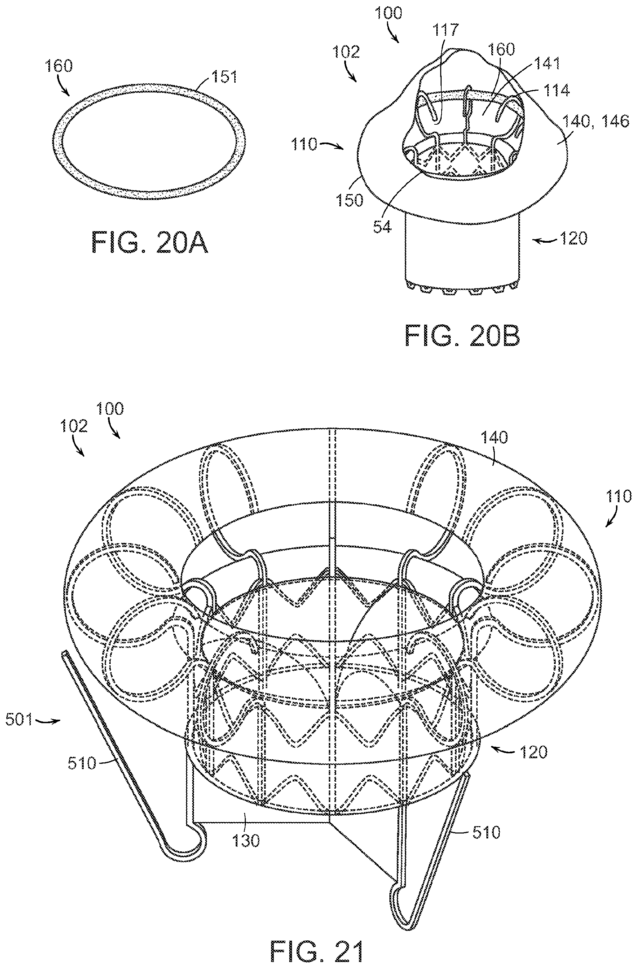

Figures 20A-20B are isometric views of a retainer support ring 160 and the prosthetic heart valve device 100 having the retainer support ring 160 in accordance with an additional embodiment of the present technology. As shown in Figure 20A, the retainer support ring 160 can be a circular-shaped ring element that has a ring circumference 151 approximately similar to a desired circumference 150 of the retainer 110 when the device 100 is in the expanded configuration 102. In another embodiment, not shown, the support ring 160 can have a different shape (e.g., oval, D-shaped, irregular, etc.) such that the support ring 160 can be configured to encourage the retainer 110 into the different shape. In one embodiment, the support ring 160 can be formed from a shape memory material (e.g., nitinol) that can collapse in a delivery configuration (not shown) to fit within a delivery catheter, and to expand toward the ring circumference 151 when the device 100 is deployed at the target location at or near the native heart valve. In other embodiments, the retainer support ring 160 may be a solid, coiled, or woven wire or band of a flexible, resilient material (e.g., biocompatible polymers or metals) with the desired degree of rigidity.

In Figure 21B, the sealing member 140, such as the sleeve 146, is pulled away for clarity only to expose the retainer support ring 160 disposed within the inner surface 141 of the retainer 110. For example, the support ring 160 can be configured to be disposed in the openings 117 of the C-shaped ribs 114 of the retainer 110 to provide additional circumferential support for the retainer 110, enhance radial rigidity and to resist and distribute distorting forces exerted on the retainer 110 during and after delivery of the device 100.

### Prosthetic Heart Valve Devices Having Stabilizing Members

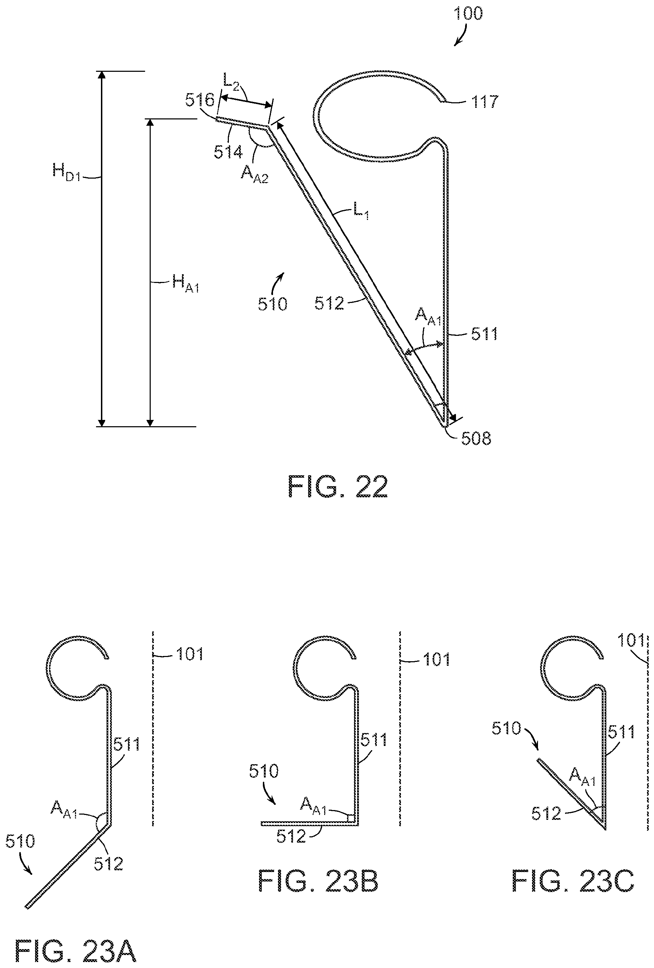

Figure 22 illustrates one embodiment of the prosthetic heart valve device 100 in an expanded configuration 102 that further comprises one or more stabilizing members 501 to help stabilize the device 100 at the native valve site and, in some embodiments, prevent tilting or lateral migration, or to inhibit upstream or downstream migration of the device 100. In some embodiments, the stabilizing members 501 may comprise one or more arms 510 extending from a lower or downstream end 123 of the valve support 120, or from the commissure posts 158. In another embodiment, the arms 510 can be configured to extend from a downstream end of rib posts 88 (shown in Figure 22). The arms 510 are configured to engage the native tissue, e.g. the valve leaflets, subannular tissue, or ventricular wall, either inside or outside the native leaflets, depending on the configuration.

Figure 22 is an enlarged schematic, side view of a prosthetic heart valve device 100 having an extended arm in accordance with an embodiment of the present technology. As shown in Figure 22, an individual arm 510 may comprise an arm body 512, an arm extension 514, and an arm tip 516. The arm body 512 has an arm body length L₁ and may connect to a post 511 at a first joint 508. The post 511 can be a valve support post 122, a retainer rib post 88, and/or another feature of the device 100 (e.g., a commissure post 158). In one embodiment, the arm body 512 may be welded, bonded, crimped, or otherwise mechanically attached to the post 511 the first joint 508. Alternatively, arms 510 may be integrally formed with posts 511, such as the valve support posts 122 or the rib posts 88. A first arm angle A_{A1} is formed by the intersection of the axes of post 511 and the arm body 512 and is selected such that the arm 512 is positionable so that the tip 516 can engage the native tissue at a desired location, e.g. the subannular tissue or ventricular wall behind the native leaflets. Figures 23A-23C are enlarged partial side views of a prosthetic heart valve device 100 having arms 510 coupled to the device at various angles with respect to a longitudinal axis 101 of the device 100 in accordance with further embodiments of the present technology. In one embodiment, the first arm angle A_{A1} can be about 10° to about 45°. In other embodiments, the first arm angle A_{A1} can be an obtuse angle (Figures 23A), generally perpendicular or approximately a 90° angle (Figure 23B), or an acute angle (Figure 23C).

Referring back to Figure 22, the arm body 512 can connect to the arm extension 514 at a distal end of the arm body 512. The arm extension 514 can have an arm extension length L₂ which can be selected or optimized for penetrating a desired distance into the native tissue, such as about 0.5-2 mm. The arm extension 514 can extend from the arm body 212 at second arm angle A_{A2}. The second arm angle A_{A2} can be formed by the intersection between the arm extension 514 and arm body 512 and be selected to provide the desired angle of engagement with the native tissue, such as about 100° to about 135°. In other embodiments, the arm extension 514 may be parallel or collinear with the arm body 512 (not shown), or may be eliminated entirely. The arm extension 514 terminates at the arm tip 516. In embodiments without an arm extension 514, the arm tip 516 can be the most distal portion of the arm body 512 (not shown).

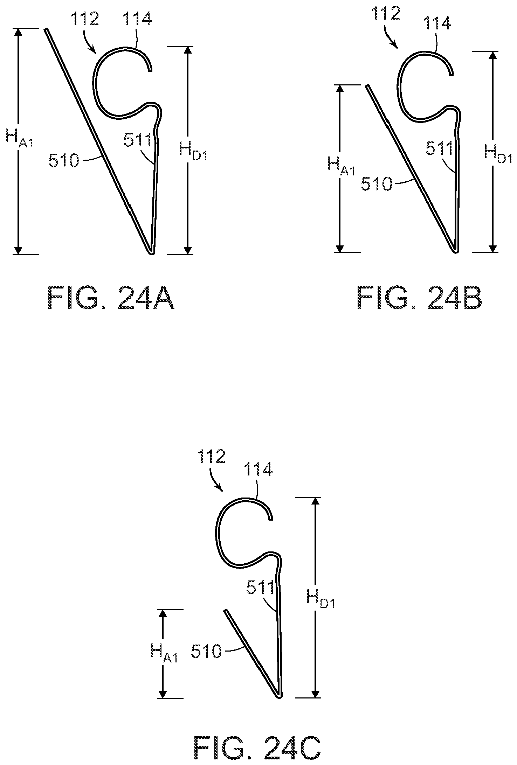

The arm 510 may have an arm height H_{A1} extending from the first joint 508 to the most distal reaching point of the arm, which could be the arm tip 516 (shown in Figure 22) along an axis parallel to the longitudinal axis 101 of the device 100. The arm height H_{A1} can be selected or optimized such that the arm tip 516 engages a desired location in the subannular anatomy when the device 100 is in a desired longitudinal position relative to the native mitral valve (e.g., when the retainer 110 is in engagement with the subannular tissue). The arm height H_{A1} will depend upon of the overall height of the retainer 110 and/or valve support 120 as well as the location of the joint 508. Figures 24A-24C are enlarged, partial side views of prosthetic heart valve devices having arms 510 of various lengths (L₁ + L₂), and accordingly having variable heights H_{A1}. As shown, the arm height H_{A1} may be greater than the overall height H_{D1} of the device 100 (represented by the post 511 and rib 114) (Figure 24A), be intermediate between the respective heights H_{D1}, H_{V1} of the retainer 110 (represented by the tissue engaging portion 112 of the rib 114) and the valve support 120 (represented by post 511) (Figure 24B), or be less than the overall height H_{D1} of both the retainer 110 (represented by rib 114) and the valve support 120 (Figure 24C).

Additional details and embodiments regarding the structure and attachment of arms or other stabilizing members suitable for use with the device 100 can be found in International PCT Patent Application No. PCT/US2012/043636, entitled "PROSTHETIC HEART VALVE DEVICES AND ASSOCIATED SYSTEMS AND METHODS," filed June 21, 2012, the entire contents of which are incorporated herein by reference.

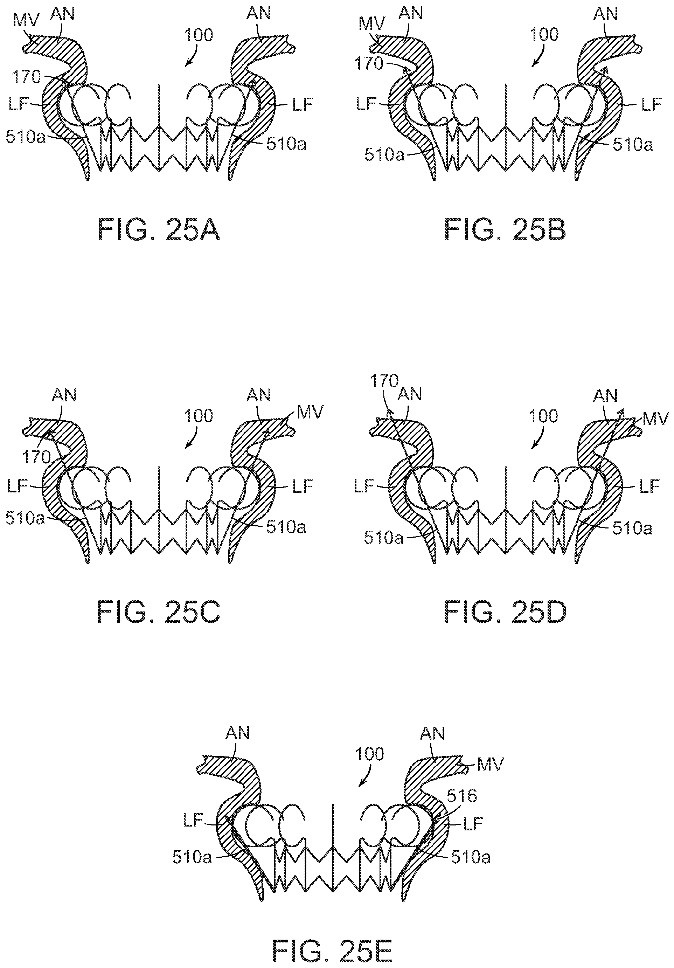

Figures 25A-25E are cross-sectional views of a heart with an implanted prosthetic heart valve device 100 having arms 510a disposed on an inward-facing surface of the leaflets LF. The embodiments of prosthetic heart valve devices 100 illustrated in Figures 25A-25E have arms 510a configured to expand to a position radially inside the leaflets LF, radially outside the leaflets LF, or a combination of inside and outside the leaflets LF. For example, Figure 25A shows the arms 510a expanding and engaging an inward surface of the leaflets LF and shows the arms 510a partially piercing the leaflets LF. In another example illustrated in Figure 25B, the arms 510a may fully penetrate the leaflets LF. In a further example, the device 100 can incorporate arms 510a that 1) completely penetrate the leaflets LF and 2) partially pierce subannular tissue (Figure 25C). Referring to Figure 25D, the device 100 can be configured to incorporate arms 510a that fully penetrate both the leaflets LF and the annular tissue of the mitral valve MV. In an additional example, Figure 25E shows the arms 510a radially engaging a greater length of the leaflet LF along the arm 510a as well as optionally piercing the leaflet LF and/or annular tissue AN at the arm tip 516. In some embodiments, all or a portion of the arms 510a may have a curvature or other suitable shape which allows the leaflets LF to conform to the outer surface of the arms 510a.

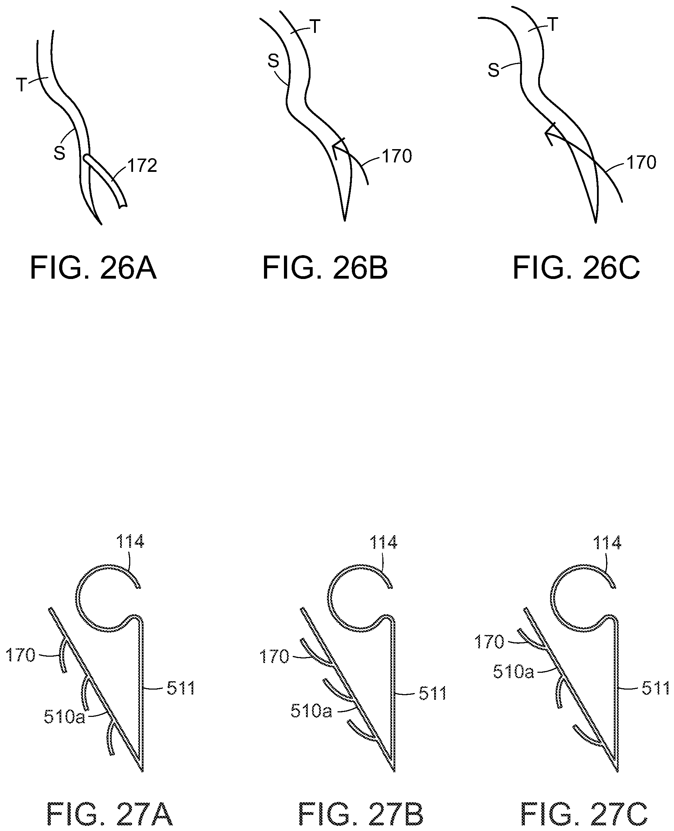

Figures 26A-26C are schematic views illustrating various embodiments of tissue engaging elements 170 for use with prosthetic heart valve devices 100 in accordance with the present technology. Tissue engaging elements 170 can include any feature that engages tissue in an atraumatic manner, such as a blunt element, or which partially pierces or fully penetrates cardiac tissue, such as a barb or spike. As used herein, "tissue engaging" refers to an element 170 which exerts a force on the tissue T but does not necessarily pierce the tissue T, such as being atraumatic to the tissue T, as shown in Figure 26A. As used herein, "partially piercing" refers to a tissue engaging feature 170 which at least partially penetrates the tissue T but does not break through an opposite surface S, as shown in Figure 26B. As used herein, "fully piercing" refers to a tissue engaging feature 170 which can both enter and exit the tissue T, as shown in Figure 26C. "Piercing" alone may refer to either partial or full piercing. Tissue engaging elements 170 may take the form of spikes, barbs, or any structure known in art capable of piercing cardiac tissue, or alternatively, any blunt or atraumatic feature configured to apply pressure on the cardiac tissue without piercing the tissue. Further details on positioning of such elements are described herein.

Figures 27A-27C are enlarged, partial side views of a prosthetic heart valve device 100 having arms 510a with tissue engaging elements 170 configured to engage an inward-facing surface of the leaflets in accordance with various embodiments of the present technology. As illustrated in Figures 27A-27C, tissue engaging elements 170 can be incorporated on and extend from the arms 510a in either a downstream direction (Figure 27A), upstream direction (Figure 27B), or in both the downstream and upstream directions (Figure 27C). In other embodiments, the tissue engaging elements 170 can be incorporated on and extend from the components of the retainer 110 and/or the valve support 120 in either or both the upstream and downstream directions.

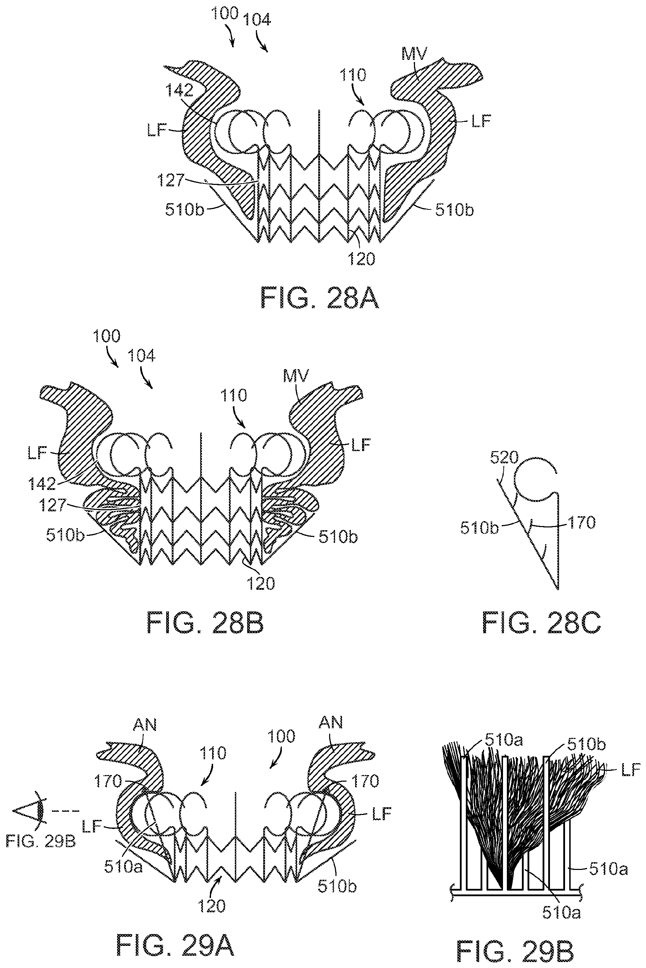

Figures 28A-28B are side views showing prosthetic heart valve devices 100 implanted at a mitral valve MV (illustrated in cross-section) in a deployed configuration 104, wherein the devices have arms 510b for engaging an outward-facing surface of the native leaflets LF in accordance with various embodiments of the present technology. Figure 28A shows an embodiment of the device 100 that includes arms 510b configured to reach behind the leaflets LF such that the leaflets LF are effectively sandwiched between the arms 510b and the outer surface 142 of the retainer 110 and/or the exterior surface 127 of the valve support 120. In another embodiment, and as shown in Figure 28B, the arms 510b may cause leaflets LF to fold upon themselves in the space between the arms 510b and the outer surface 142 of the retainer 110 and/or the exterior surface 127 of the valve support 120. Figure 28C is an enlarged, partial side view of a prosthetic heart valve device 100 having the arm 510b with tissue engaging elements 170 configured to engage an outward-facing surface of the leaflets in accordance with various embodiments of the present technology. As shown in Figure 28C, the arm 510b includes tissue engaging elements 170 on an inside surface 520 of the arm 510b such that they are oriented toward the leaflet tissue.

In accordance with another embodiment of the present technology, Figure 29A is a side view showing a prosthetic heart valve device 100 implanted at a mitral valve MV (illustrated in cross-section). The device shown in Figure 29A has arms 510b for engaging an outward-facing surface of the native leaflets LF and arms 510a for engaging an inward-facing surface of the native leaflets LF. Inside/outside arms 510a, 510b may further comprise tissue engaging elements 170 on a radially inside surface or radially outside surface of the arms 510a, 510b, respectively, for engaging or piercing the leaflet tissue. The arrangement of inside/outside arms 510a, 510b around a circumference of the device 100 can alternate in a pre-designed pattern. For example, inside arms 510a can alternate with outside arms 510b as shown in Figure 29B, or alternatively, arms 510a, 510b may extend radially outward and/or radially inward randomly or at irregular intervals, depending on placement of the device 100 and with respect to alignment with the native posterior and anterior leaflets.

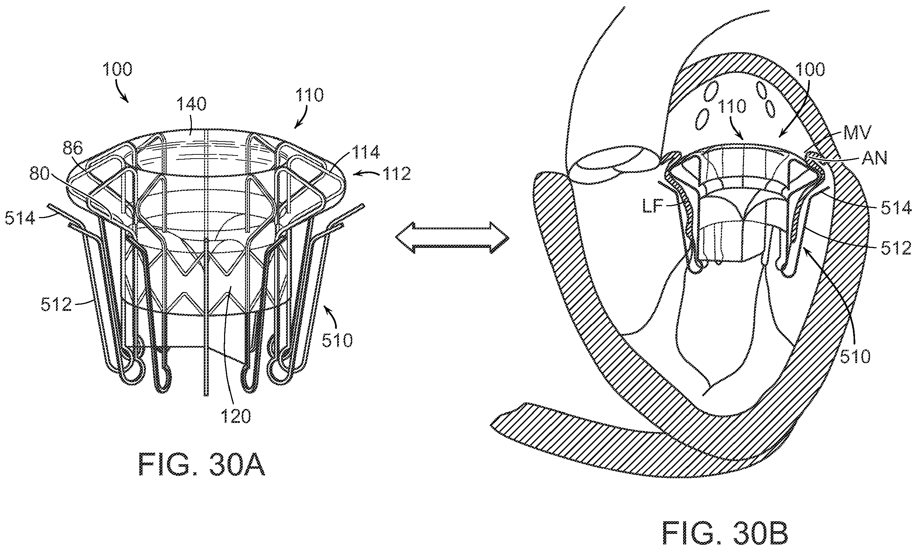

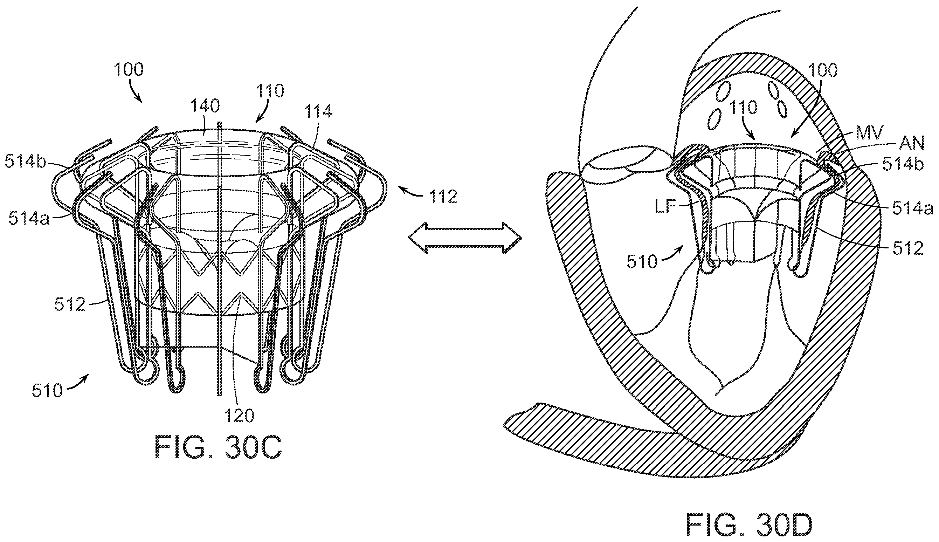

Figures 30A and 30C are isometric views of the prosthetic heart valve device 100 having arms 510 with a similar profile as a profile of the retainer 110, and Figures 30B and 30D are side views of the prosthetic heart valve devices 100 of Figures 30A and 30C, respectively, and shown implanted at a mitral valve (illustrated in cross-section) in accordance with another embodiment of the present technology. As shown in Figure 30A, the arms 510 can have a similar overall profile as a profile of the retainer 110. The retainer 110 can include ribs 114 having varying shapes, sizes and/or outwardly or inwardly oriented tissue engaging portion segments 80, 86 for forming the overall retainer 110 profile. Accordingly, the arms 510 can also have varying shapes, sizes and/or outwardly or inwardly oriented arm segments that mimic the retainer 110 profile. In the embodiment shown in Figures 30A-30B, the arms 510 are configured to clamp leaflets LF and/or the annulus AN tissue between the arms 510 and the tissue engaging portion 112 of the ribs 114 so as to conform the leaflet tissue to the shape of the retainer 110 for enhanced sealing and anchoring of the device 100. For example, Figure 30A illustrates one embodiment in which the arm extensions 514 and/or the arm bodies 512 may partially mimic the shape of the ribs 114 and/or the tissue engaging portion segments 80, 86.

Figures 30C-30D illustrates another embodiment in which first and second arm extensions 514a and 514b and/or arm bodies 512 more closely follow the shape of the ribs 114. For example, the arms 510 can include the arm body 512 and multiple arm extensions (e.g., first arm extension 514a and second arm extension 514b) that are configured to clamp leaflets LF and/or the annulus AN tissue between the arms 510 and the tissue engaging portion 112 of the ribs 114 so as to conform the leaflet tissue to both lower and upper regions of the tissue engaging portion 112 for enhanced sealing and anchoring of the device 100. Embodiments encompassed by Figures 30A-30D can apply to outward surface engaging arms 510b and/or inward surface engaging arms 510a.

In some embodiments, the prosthetic heart valve device 100 may incorporate a plurality of arms 510 around a circumference of the device 100; however, in other embodiments, the device 100 may include the plurality of arms in groupings (e.g., first and second groupings so as to engage the posterior and anterior leaflets, respectively). Additionally, the arms 510 may extend from the retainer 110 and/or valve support 120 independently of other components including other arms 510, such as shown in Figure 31A. In other embodiments and as shown in Figure 31B, the device 100 may further include at least one first arm 510x interconnected with at least one second arm 510y by interconnecting arm struts 522. The arm struts 522 can be configured to be circumferentially expandable and may connect all arms 510 (e.g., arm 510x and 510y) or one or more groups of arms 510. In some embodiments, the arm struts 522 can limit the outward extension of the arms 510x, 510y away from the device 100.

In accordance with aspects of the present technology, the arms 510 can be coupled to and/or extend from components of the device 100 symmetrically and/or asymmetrically around the circumference 150 of the device 100. Figures 32A-32D are schematic top views of arm location patterns with respect to the ribs 114 of the retainer 110 (e.g., as shown in Figure 31A). The arms 510 can be interspersed with ribs 114 (Figures 32A and 32C), in the same radial plane as the ribs 114 of the retainer 110 (Figure 32B), or both interspersed and in plane with the ribs 114 (Figure 32D). Further, the arms 510 may be configured to extend outside the expanded outer circumference 150 of the retainer 110 (Figure 32B), inside the expanded outer circumference 150 of the retainer 110 (Figure 32A), extend to the same outer circumference 150 of the retainer 110 (Figure 32C), or a combination of these configurations (Figure 32D).

In the above-described embodiments, the arms 510 may be configured to engage tissue independently of the deployment of retainer 110. For example, delivery catheters suitable for the delivery of the prosthetic heart valve devices 100 may be equipped with separate mechanisms operable to deploy the arms 510 and the retainers 110 individually or otherwise independently of each other. In this way, the retainer 110 may be first released into engagement with the native tissue so that the position of device 100 may be assessed and adjusted by the operator until the desired final position has been attained. Following deployment and positioning of the retainer 110, the arms 510 can be released to engage the tissue. Such deployment systems and methods are useful when the arms 510 are equipped with tissue engaging elements 170 which, once deployed, may prohibit any repositioning of the device 100. In some embodiments, the retainer 110 will be equipped with atraumatic tissue engagement elements 170 which do not penetrate tissue or inhibit device relocation once the retainer 110 has been deployed. Accordingly, some embodiments of the device 100 may be repositionable even with the retainer 110 expanded so long as the arms 510 are constrained in an undeployed configuration, with the device 100 becoming permanently anchored only when the arms 510 are released.

Alternatively or in addition to tissue engaging elements 170 present on the arms 510 as described above, tissue engaging elements 170 may be present on other components of the device 100. Figures 33A-33E are side views of prosthetic heart valve devices 100 having tissue engaging elements 170 on varying structures of the device 100 in accordance with additional embodiments of the present technology. Figure 33A shows tissue engaging elements 170 incorporated on the tissue engaging portion 112 of the ribs 114 of the retainer 110. Figure 33B illustrates an embodiment of the device 100 having the tissue engaging elements 170 along the struts 124 of the valve support 120. Likewise, Figure 33C shows an embodiment of the device 100 having the tissue engaging elements 170 along the posts of the valve support 120. In another embodiment, shown in Figure 33D, the tissue engaging elements 170 can be incorporated along the surfaces of several device components, such as the ribs 114 as well as the posts 122 and struts 124 of the valve support 120.

The tissue engaging elements 170 are shown in Figures 33A-33D schematically, but one of ordinary skill in the art will recognize that the elements can be any of a variety of tissue engaging elements 170 described herein (e.g., atraumatic, partially piercing, fully penetrating, etc.), or in other embodiments, a combination of different types of tissue engaging elements 170. Additionally, the tissue engaging elements 170 are shown oriented in an upstream direction (e.g., to inhibit upstream migration of the device 100) in Figures 33A-33B; however, in other embodiments, the tissue engaging elements 170 can be oriented in a downstream direction (e.g., to inhibit downstream migration of the device 100), or in a combination of downstream and upstream oriented directions (shown in Figures 33C-33D). The tissue engaging elements 170 can be incorporated symmetrically around a circumference or outside surface of the device 100, or in other embodiments, the tissue engaging elements 170 can be incorporated asymmetrically. For example, in some embodiments, the tissue engaging elements 170 can be present on a side of the device 100 aligned with the posterior leaflet, but be absent or have a different arrangement on a side of the device 100 aligned with the anterior leaflet such that the wall separating the aortic valve from the left ventricle will not be affected by the tissue engaging elements 170.

Figure 33E illustrates an embodiment of the device 100 having tissue engaging elements 170, such as spikes on a rib tip 117 of the rib 114, wherein the spikes 174 can be configured to fully or partially penetrate subannular tissue when the device 100 is deployed on or under the annulus of the mitral valve. In some embodiments, the tissue engaging elements 170 (e.g., spikes) can include barbs 176 or other features for retaining the tissue engaging elements 170 (e.g., spikes) in the tissue. In other embodiments, the tissue engaging elements 170 (e.g., spikes) can be blunt so as to engage but not penetrate the subannular tissue. Figures 33F-33G are enlarged side views of tissue engaging elements 170 (e.g., hooks, spikes, etc.) suitable for use on rib tips 117 of the ribs 114. In one embodiment, shown in Figure 3F, the rib tip 117 may include a rounded hook 172 that may partially pierce other fully penetrate cardiac tissue at the target location with the retainer 110 is deployed. In another embodiment, shown in Figure 33G, the rib tip 117 may include a barbed protrusion such as a spike 174, 176 for piercing cardiac tissue at the target location.

Alternatively, tissue engaging elements 170, such as bumps, ridges, or other protrusions configured to exert frictional forces on cardiac tissue, may be also present on one or more valve support struts 124, valve support posts 122, and/or other components (e.g., sealing members 140). These tissue engaging elements 170 can be disposed on an outer portion of these features and can be configured to extend outwardly to engage the native leaflets and to stabilize and firmly anchor the device 100 in the desired location. Alternatively, ridges, scales, bristles, or other features having directionality may be formed on the surface of the ribs 114 or sealing member 140 to allow movement relative to native tissue in one direction, while limiting movement in the opposite direction.

In accordance with another embodiment of the prosthetic treatment device 100, tissue engaging elements 170 can be incorporated into sealing members 140 (e.g., sleeve 146). Figures 34A-34B are an isometric view and an enlarged detail view of a prosthetic heart valve device 100 having a sealing member 140 configured with tissue engaging elements 170. Referring to Figures 34A-34B together, the tissue engaging elements 170 can comprise metallic or polymeric wires 178 or fibers, rigid and sharp enough to penetrate tissue, which are woven into or otherwise coupled to sealing member 140 materials. The sealing member 140 can then be attached to outer and/or inner surfaces 141, 142 of the retainer 110 and/or interior and/or exterior surfaces 126, 127 of the valve support 120 such that tissue engaging elements 170 extend radially outward from the sealing member 140 to engage the adjacent leaflets or other tissue.

Figures 35A-35F are enlarged side views of embodiments of additional tissue engaging elements that can be incorporated on various device structures (referred collectively as "ST"), such struts, posts, arms, and/or ribs which may be incorporated into device features, such as the retainer 110 or valve support 120. For example, the additional tissue engaging elements may comprise one or more cut-out protrusions 350 (Figures 35A and 35B) in place of or in addition to tissue engaging elements 170. In a collapsed or straightened configuration, as shown by the side view of Figure 35C, cut-out protrusion 350 maintains low relief relative to the surface of structure ST to maintain a low profile during delivery. As the device 100 expands and structure ST changes to its deployed configuration (e.g. a curvature as shown in Figure 35D), the protrusion separates from the ST to a higher relief. The protrusion 350 may also be configured to grab subannular tissue, pulling the cut-out protrusions even farther away from structure ST. The device structures ST may also be shaped to include sharp protrusions 352 along one or more of its edges or faces, as illustrated in Figure 35E, or may also include pointed scale-like protrusions 354, as shown in Figure 35F.

### Prosthetic Heart Valve Devices Having Atrial Extension Members

Figure 36A is an isometric view of a prosthetic heart valve device 100 having an atrial extension member 410 in accordance with various embodiments of the present technology. The atrial extension member 410 can be generally cylindrical, being formed around the longitudinal axis 101 of the device 100 with a circular, oval, elliptical, kidney-shaped or other suitable cross-section. As shown in Figure 36A, the atrial extension member 410 can be coupled to the retainer ribs 114, to the posts 122 of the valve support 120, or to some other device component. In one embodiment, the atrial extension member 410 can be formed by extension 411 of the ribs 114 in an upward direction. The atrial extension member 410 can include an upstream portion 412 formed by the extension 411 of the ribs 114 and including interconnecting struts 414 and/or other posts which can be arranged in a variety of geometric patterns (e.g., chevron, diamond, etc.) for support and resilience of the atrial extension member 410. The atrial extension member can be configured to extend into an intra-annular, supra-annular or atrial location in the heart to provide additional support to the device 100 and/or prevent the device 100 from moving in a downstream or upstream direction. A sealing member 140, such as the sleeve 146, can optionally reside on the inner 420 and/or outer 422 surface of the atrial extension member 410.

Figures 36B-36C are schematic, top views of an embodiment of a prosthetic heart valve device 100 having an atrial extension member 410 without (Figure 36B) and with (Figure 36C) a twisting force applied to the device 100 in accordance with the present technology. Figure 36B shows the device 100 in the expanded configuration 102 having the atrial extension member 410 and a plurality of ribs 114 positioned circumferentially around the device 100 to form the retainer 110. Figure 36B shows the device 100 in a deployed configuration 104 wherein a twisting force T_{w} is applied to the retainer 110 such that the ribs 114 are flexed, bent and/or rotated with respect to an outer surface 430 of the device 100 and/or the atrial extension member 410 to conform to the native heart valve tissue (e.g., mitral valve annulus).

The expandable retainer, valve support, arms, atrial extension may be made from any number of suitable biocompatible materials, e.g., stainless steel, nickel-titanium alloys such as Nitinol^{™}, various polymers, ELGILOY^{®} (Elgin, IL), pyrolytic carbon, silicone, polytetrafluoroethylene (PTFE), or any number of other materials or combination of materials depending upon the desired results. The arm members may also be coated or covered with a material that promotes tissue in-growth, e.g., Dacron^{®}, PTFE, coatings, etc.

### Delivery Systems

Figures 37A-37D illustrate one embodiment of a delivery system 10 suitable for delivery of the prosthetic heart valve devices disclosed herein. As used in reference to the delivery system, "distal" refers to a position having a distance farther from a handle of the delivery system 10 along the longitudinal axis of the system 10, and "proximal" refers to a position having a distance closer to the handle of the delivery system 10 along the longitudinal axis of the system10.

Figure 37A illustrates one embodiment of the delivery system 10 which may be used to deliver and deploy the embodiments of the prosthetic heart valve device 100 disclosed herein through the vasculature and to the heart of a patient. The delivery system 10 may optionally include a guiding catheter GC having a handle 12 coupled to a delivery shaft 16, which in one embodiment is 34F or less, and in another embodiment, 28F or less in diameter. The guiding catheter GC may be steerable or pre-shaped in a configuration suitable for the particular approach to the target valve. The delivery catheter 18 is placed through a hemostasis valve HV on the proximal end of guiding catheter GC and includes a flexible tubular outer shaft 19 extending to a delivery sheath 20 in which the device 100 is positioned in a collapsed or delivery configuration 106. A flexible inner shaft 28 is positioned slideably within outer shaft 19 and extends through the device 100 to a nosecone 21 at the distal end. The inner shaft 28 has a guidewire lumen through which a guidewire 24 may be slideably positioned. The device 100 is coupled to the inner shaft 28 and is releasable from the inner shaft 28 by release wires 30, as more fully described below. The delivery sheath 20 can protect and secure the device 100 in its collapsed configuration 106 during delivery. The outer shaft 20 is coupled to a retraction mechanism 23 on the handle 14 of the delivery catheter 18. Various retraction mechanisms 23 may be used, such as an axially-slidable lever, a rotatable rack and pinion gear, or other known mechanisms. In this way, the outer shaft 20 may be retracted relative to the inner shaft 28 to release (e.g., deploy) the device 100 from the sheath 20.

Figure 37B shows the distal end of the delivery catheter 18 with the sheath 20 cut away to illustrate the coupling of the device 100 to the inner shaft 28. A plurality of locking fingers 32 are coupled to the nose cone 21 and extend proximally through the interior of the valve support 120 of the device 100. As shown in Figure 37C, a selected number of posts 122 of the valve support 120 have a coupling element 61 comprising a tab 34 cut out from each post 122 at a proximal end thereof. The tab 34 may be deflected inwardly from the post 122 as shown in Figure 37B and is configured to extend through a window 42 in the locking finger 32 as shown in Figure 37D. The release wires 30 pass through the holes 40 in the tabs 34, which prevents the tabs 34 from being withdrawn from the windows 42 to secure the device 100 to the inner shaft 28. The pull-wires 30 can be sandwiched tightly between the tabs 34 and the locking fingers 32, such that friction temporarily prevents the pull-wire 30 from slipping in a proximal or distal direction. In this way, the sheath 20 may be retracted relative to the device 100 to permit expansion of the device 100 while the inner shaft 28 maintains the longitudinal position of the device 100 relative to the anatomy. The pull-wires 30 may extend proximally to the handle 14, for example, in between the inner shaft 28 and the outer shaft 19 or within one or more designated lumens. A suitable mechanism (not shown) on the handle 14 can allow the operator to retract the release wires 30 in a proximal direction until they are disengaged from the tabs 34. Accordingly, the device 100 can be released from the locking fingers 32 and expand for deployment at the target site.

Figures 38A-38D are schematic, cross-sectional side views of a heart H showing a trans-septal or antegrade approach for delivering and deploying a prosthetic heart valve device 100. As shown in Figure 38A, a guidewire 24 may be advanced intravascularly using any number of techniques, e.g., through the inferior vena cava IVC or superior vena cava SVC, through the inter-atrial septum IAS and into the right atrium RA. The guiding catheter GC may be advanced along the guidewire 24 and into the right atrium RA until reaching the anterior side of the atrial septum AS, as shown in Figure 38B. At this point, the guidewire 24 may be exchanged for the needle 25, which is used to penetrate through the inter-atrial septum IAS (Figure 38C). The guiding catheter GC may then be advanced over the needle 25 into the left atrium LA, as shown in Figure 38D. The guiding catheter GC may have a pre-shaped or steerable distal end to shape or steer the guiding catheter GC such that it will direct the delivery catheter 18 (Figure 37A) toward the mitral valve.

As an alternative to the trans-septal approach, the mitral valve may also be accessed directly through an incision in the left atrium. Access to the heart may be obtained through an intercostal incision in the chest without removing ribs, and a guiding catheter may be placed into the left atrium through an atrial incision sealed with a purse-string suture. A delivery catheter may then be advanced through the guiding catheter to the mitral valve. Alternatively, the delivery catheter may be placed directly through an atrial incision without the use of a guiding catheter.

Figures 39A-39C are cross-sectional views of the heart illustrating a method of implanting a prosthetic heart valve device 100 using a trans-septal approach. Referring to Figures 39A-39C together, the distal end 21 of the delivery catheter 18 may be advanced into proximity to the mitral valve MV. Optionally, and as shown in Figure 39A, a guidewire GW may be used over which catheter 18 may be slideably advanced over a guidewire GW. The sheath 20 of the delivery catheter 18, which contains the device 100 in a collapsed configuration 106, is advanced through the mitral valve annulus AN between native leaflets LF, as shown in Figure 39A. Referring to Figure 39B, the sheath 20 is then pulled back proximally relative to the distal nose cone 21 allowing the device 100 to expand such that retainer 110 pushes the leaflets LF outwardly to fold beneath the mitral valve annulus AN. After the sheath 20 has been removed and the device 100 allowed to expand, the delivery system can still be connected to the device 100 (e.g., system eyelets, not shown, are connected to the device eyelets) so that the operator can further control the placement of the device 100 as it expands toward the expanded configuration 102. For example, the device 100 may be expanded upstream or downstream of the target location then pushed downstream or upstream, respectively, into the desired target location before releasing the device 100 from delivery system 10. Once the device 100 is positioned at the target site, the pull-wires 30 (Figures 37A-37B) may be retracted in a proximal direction, to detach the device 100 in the deployed configuration 104 from the delivery catheter 18. The delivery catheter 18 can then be removed as shown in Figure 39C. Alternatively, the device 100 may not be connected to the delivery system 10 such that the device 100 deploys and is fully released from the delivery system 10.

Figures 40A-40C illustrate delivery of the device 100 in the collapsed configuration 106 to the mitral valve MV via a trans-apical approach. Referring to Figure 40A, the delivery catheter 18 is advanced through a guiding catheter GC that has been inserted into the left ventricle of the heart through a puncture in the left ventricle wall at or near the apex of the heart. The catheter can be sealed by a purse-string suture. Alternatively, the delivery catheter 18 may be placed directly through a purse-string-sealed trans-apical incision without a guiding catheter. The sheath 20 and the device 100 (e.g., in the collapsed configuration 106) within the sheath 20 are advanced through the mitral annulus AN between native leaflets LF as shown in Figure 40A. Referring to Figure 40B, the sheath 20 is pulled proximally such that the device 100 expands to the expanded and/or deployed configurations 102, 104. The delivery system 10 can remain connected to the device 100 (e.g., system eyelets, not shown, are connected to the device eyelets) after removing the sheath 20 so that the operator can control the placement of the device 100 while the device expands toward the expanded configuration 102. The pull-wires 30 may be retracted in a proximal direction to release the device 100 from the delivery system 10, allowing the delivery system 10 to be removed and the device to be fully implanted at the mitral valve MV in the deployed configuration 104. In one embodiment, the device 100 may be expanded upstream or downstream of the desired target location then pulled or pushed downstream or upstream, respectively, into the target location before releasing the device 100 from delivery system 10. Alternatively, the device 100 may not be connected to the delivery system 10 such that the device 100 deploys and is fully released from the delivery system 10.

In another embodiment, not shown, the device 100 can be mounted on an expandable balloon of a delivery catheter and expanded to its functional size by inflation of the balloon. When using a balloon delivery system, the device 100 can be advanced from the delivery shaft to initially position the device in a target location. The balloon can be inflated to fully expand the device 100. The device 100 may then be adjusted using the device locking hub to position the device into the desired implantation site (e.g., just below the annulus of the native mitral valve). In another embodiment, the balloon initially can be partially inflated to partially expand the valve assembly in the left atrium. The delivery system 10 can then be adjusted to push or pull (depending on the approach) the partially expanded valve into the implantation site, after which the valve assembly can be fully expanded to its functional size.

### Additional Embodiments

Features of the prosthetic heart valve device components described above and illustrated in Figures 10A-40C can be modified to form additional embodiments configured in accordance with the present technology. For example, the prosthetic heart valve device 100 illustrated in Figure 18 and other prosthetic heart valve devices described above without stabilizing members can include stabilizing members, such as arms that are coupled to the valve support or other feature and are configured to extend radially outward to engage leaflet tissue. Similarly, any of the prosthetic heart valve devices described above and illustrated in Figures 10A-40C can include features such as sealing members as well as stabilizing features and tissue engaging elements. Features of the prosthetic heart valve device components described above also can be interchanged to form additional embodiments of the present technology.

### Conclusion

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A prosthetic heart valve device for treating a mitral valve, the prosthetic heart valve device comprising:
an expandable retainer (110) configured to engage cardiac tissue at or downstream of a native mitral valve annulus; and
a valve support (120) coupled to and extending in a downstream direction from the expandable retainer (110), wherein the valve support (120) is configured to support a prosthetic valve (130), wherein the valve support includes a plurality of posts (122) connected circumferentially by a plurality of struts (124);
wherein the retainer (110) includes a plurality of flexible arcuate ribs (114) extending outward from the valve support (120) and in an upstream direction, the ribs being distributed around a perimeter of the valve support,
wherein the expandable retainer (110) is configured to conform to the shape of the native mitral valve annulus while the valve support (120) remains substantially unchanged,
**characterized in that** the prosthetic heart valve device is configured to be delivered intravascularly to a desired location in the heart while being in a delivery configuration within a delivery catheter.

2. The device of claims 1 wherein the device is moveable into a plurality of configurations including:
a first configuration in which the valve support (120) and the retainer (110) are radially contracted, and wherein the valve support (120) has a first cross-sectional shape;
a second configuration in which the valve support (120) and the retainer (110) are radially expanded, and wherein the valve support (120) has a second cross-sectional shape greater than the first cross-sectional shape; and
a third configuration in which the retainer (110) is engaged with and at least partially deformed by tissue on or near the annulus while the valve support (120) remains in the second cross-sectional shape.

3. The device of claim 2 wherein the retainer (110) has an outer circumference, the outer circumference being generally circular in the second configuration and generally non-circular in the third configuration.

4. The device of claim 3 wherein the retainer (110) has an inner circumference, the inner circumference defining a passage for blood to flow through the valve support, and wherein the inner circumference is substantially circular in the third configuration.

5. The device of claim 1 wherein the ribs (114) are non-symmetrically distributed around the perimeter.

6. The device of claim 1 wherein the ribs (114) are symmetrically distributed around the perimeter.

7. The device of claim 1 wherein the arcuate ribs (114) have rib tips (117) that are oriented inwardly toward the longitudinal axis.

8. The device of any one of preceding claims, further comprising a sealing member (140) disposed on a surface of the retainer (110) and configured to seal against at least the tissue on or near the annulus to inhibit blood flow between the retainer (110) and the tissue.

9. The device of any one of preceding claims wherein the retainer (110) is self-expanding.

10. The device of any one of preceding claims wherein the retainer (110) comprises nitinol.

11. The device of any one of preceding claims:
the plurality of flexible ribs (114) being coupled to the posts; and
the posts are more rigid than the ribs (114).

## Patentansprüche

1. Prothetische Herzklappenvorrichtung zum Behandeln einer Mitralklappe, wobei die prothetische Herzklappenvorrichtung umfasst:
einen expandierbaren Halter (110), der konfiguriert ist, um in Herzgewebe an oder stromabwärts von einem nativen Mitralklappenanulus einzugreifen; und
eine Klappenstütze (120), die mit dem expandierbaren Halter (110) gekoppelt ist und sich in einer stromabwärtigen Richtung von diesem erstreckt, wobei die Klappenstütze (120) konfiguriert ist, um eine prothetische Klappe (130) zu stützen, wobei die Klappenstütze mehrere Stifte (122) einschließt, die durch mehrere Streben (124) umlaufend verbunden sind;
wobei der Halter (110) mehrere flexible bogenförmige Rippen (114) beinhaltet, die sich von der Klappenstütze (120) nach außen und in eine stromaufwärtige Richtung erstrecken, wobei die Rippen um einen Umfang der Klappenstütze herum verteilt sind,
wobei der erweiterbare Halter (110) konfiguriert ist, um sich der Form des natürlichen Mitralklappenrings anzupassen, während die Klappenstütze (120) im Wesentlichen unverändert bleibt,
**dadurch gekennzeichnet, dass** die prothetische Herzklappenvorrichtung konfiguriert ist, um intravaskulär an eine gewünschte Stelle in dem Herzen eingeführt zu werden, während sie sich in einer Einführkonfiguration innerhalb eines Einführkatheters befindet.

2. Vorrichtung nach Anspruch 1, wobei die Vorrichtung in mehrere Konfigurationen bewegbar ist, einschließlich:
einer ersten Konfiguration, bei der die Klappenstütze (120) und der Halter (110) radial zusammengezogen sind, und wobei der die Klappenstütze (120) eine erste Querschnittsform aufweist;
einer zweiten Konfiguration, bei der die Klappenstütze (120) und der Halter (110) radial expandiert sind, und wobei der die Klappenstütze (120) eine zweite Querschnittsform aufweist, die größer als die erste Querschnittsform ist; und
einer dritten Konfiguration, bei der der Halter (110) in das Gewebe am oder in der Nähe des Anulus eingreift und zumindest teilweise durch dieses verformt wird, während die Klappenstütze (120) in der zweiten Querschnittsform verbleibt.

3. Vorrichtung nach Anspruch 2, wobei der Halter (110) einen Außenumfang aufweist, wobei der Außenumfang in der zweiten Konfiguration allgemein kreisförmig und in der dritten Konfiguration allgemein nicht kreisförmig ist.

4. Vorrichtung nach Anspruch 3, wobei der Halter (110) einen Innenumfang aufweist, wobei der Innenumfang einen Durchgang für Blut definiert, um durch die Klappenstütze zu fließen, und wobei der Innenumfang in der dritten Konfiguration im Wesentlichen kreisförmig ist.

5. Vorrichtung nach Anspruch 1, wobei die Rippen (114) unsymmetrisch um den Umfang herum verteilt sind.

6. Vorrichtung nach Anspruch 1, wobei die Rippen (114) symmetrisch um den Umfang herum verteilt sind.

7. Vorrichtung nach Anspruch 1, wobei die bogenförmigen Rippen (114) Rippenspitzen (117) aufweisen, die nach innen zur Längsachse ausgerichtet sind.

8. Vorrichtung nach einem der vorhergehenden Ansprüche, ferner umfassend ein Dichtungselement (140), das auf einer Oberfläche des Halters (110) angeordnet ist und konfiguriert ist, um gegen zumindest das Gewebe am oder in der Nähe des Anulus abzudichten, um Blutfluss zwischen dem Halter (110) und dem Gewebe zu verhindern.

9. Vorrichtung nach einem der vorhergehenden Ansprüche, wobei der Halter (110) selbstexpandierbar ist.

10. Vorrichtung nach einem der vorhergehenden Ansprüche, wobei die Halterung (110) Nitinol umfasst.

11. Vorrichtung nach einem der vorhergehenden Ansprüche:
wobei die mehreren flexiblen Rippen (114) an die Stifte gekoppelt sind; und
die Stifte starrer als die Rippen (114) sind.

## Revendications

1. Dispositif de valvule cardiaque prothétique destiné à traiter une valvule mitrale, le dispositif de valvule cardiaque prothétique comprenant :
un dispositif de retenue (110) extensible conçu pour entrer en prise avec du tissu cardiaque au niveau ou en aval d'un anneau de valvule mitrale native ; et
un support de valvule (120) accouplé à l'élément de retenue expansible (110) et s'étendant dans une direction aval depuis celui-ci, dans lequel le support de valvule (120) est conçu pour supporter une valvule prothétique (130), dans lequel le support de valvule comporte une pluralité de montants (122) reliés circonférentiellement par une pluralité d'entretoises (124) ;
dans lequel le dispositif de retenue (110) comporte une pluralité de nervures arquées (114) flexibles s'étendant vers l'extérieur à partir du support de valvule (120) et dans une direction amont, les nervures étant réparties autour d'un périmètre du support de valvule,
dans lequel le dispositif de retenue (110) extensible est conçu pour s'adapter à la forme de l'anneau de la valvule mitrale native, tandis que le support de valvule (120) reste sensiblement inchangé,
**caractérisé en ce que** le dispositif de valvule cardiaque prothétique est conçu pour être administré par voie intravasculaire à un emplacement souhaité dans le cœur lorsqu'il est dans une configuration d'administration à l'intérieur d'un cathéter d'administration.

2. Dispositif selon la revendication 1, dans lequel le dispositif est mobile dans une pluralité de configurations comportant :
une première configuration dans laquelle le support de valvule (120) et le dispositif de retenue (110) sont radialement contractés, et le support de valvule (120) ayant une première forme en section transversale ;
une deuxième configuration dans laquelle le support de valvule (120) et le dispositif de retenue (110) sont radialement déployés, et dans lequel le support de valvule (120) a une seconde forme en section transversale supérieure à la première forme en section transversale ; et
une troisième configuration dans laquelle le dispositif de retenue (110) est en prise avec et au moins partiellement déformé par un tissu sur ou près de l'anneau tandis que le support de valvule (120) reste dans la seconde forme en section transversale.

3. Dispositif selon la revendication 2, dans lequel le dispositif de retenue (110) a une circonférence extérieure, la circonférence extérieure étant généralement circulaire dans la deuxième configuration et généralement non circulaire dans la troisième configuration.

4. Dispositif selon la revendication 3, dans lequel le dispositif de retenue (110) a une circonférence intérieure, la circonférence intérieure définissant un passage pour que le sang s'écoule à travers le support de valvule, et dans lequel la circonférence intérieure est sensiblement circulaire dans la troisième configuration.

5. Dispositif selon la revendication 1, dans lequel les nervures (114) sont réparties de manière non symétrique autour du périmètre.

6. Dispositif selon la revendication 1, dans lequel les nervures (114) sont réparties de manière symétrique autour du périmètre.

7. Dispositif selon la revendication 1, dans lequel les nervures (114) arquées ont des extrémités de nervures (117) qui sont orientées vers l'intérieur vers l'axe longitudinal.

8. Dispositif selon l'une quelconque des revendications précédentes, comprenant en outre un élément d'étanchéité (140) disposé sur une surface du dispositif de retenue (110) et conçu pour se sceller contre au moins le tissu sur ou à proximité de l'anneau pour empêcher l'écoulement sanguin entre le dispositif de retenue (110) et le tissu.

9. Dispositif selon l'une quelconque des revendications précédentes, dans lequel le dispositif de retenue (110) est auto-extensible.

10. Dispositif selon l'une quelconque des revendications précédentes, dans lequel le dispositif de retenue (110) comprend du nitinol.

11. Dispositif selon l'une quelconque des revendications précédentes :
la pluralité de nervures flexibles (114) étant accouplée aux montants ; et
les montants sont plus rigides que les nervures (114).
